(19) Europäisches Patentamt
European Patent Office
Office européen des brevets

(11) Veröffentlichungsnummer : **0 052 799**
**B1**

(12) # EUROPÄISCHE PATENTSCHRIFT

(45) Veröffentlichungstag der Patentschrift : **30.01.85**

(51) Int. Cl.⁴ : **C 07 J 63/00**, A 61 K 31/16, A 61 K 31/19, A 61 K 31/215, A 61 K 31/275, A 61 K 31/34

(21) Anmeldenummer : **81109202.2**

(22) Anmeldetag : **29.10.81**

(54) **Neue D-Homosteroide, ihre Verwendung und Herstellung, sowie pharmazeutische Präparate damit.**

(30) Priorität : **21.11.80 CH 8622/80**

(43) Veröffentlichungstag der Anmeldung : **02.06.82 Patentblatt 82/22**

(45) Bekanntmachung des Hinweises auf die Patenterteilung : **30.01.85 Patentblatt 85/05**

(84) Benannte Vertragsstaaten : **AT BE CH DE FR GB IT LI LU NL SE**

(56) Entgegenhaltungen :
**EP-A- 0 013 959**
**FR-A- 2 305 976**
**US-A- 3 254 074**
**US-A- 3 705 179**
**US-A- 3 917 829**

(73) Patentinhaber : **F. HOFFMANN-LA ROCHE & CO. Aktiengesellschaft**
**CH-4002 Basel (CH)**

(72) Erfinder : **Alig, Leo, Dr.**
**Liebrütistrasse 32**
**CH-4303 Kaiseraugst (CH)**
Erfinder : **Fürst, Andor, Dr.**
**Magnolienpark 14**
**CH-4052 Basel (CH)**
Erfinder : **Keller, Peter, Dr.**
**Hinterlindenweg 53**
**CH-4153 Reinach (CH)**
Erfinder : **Müller, Marcel, Dr.**
**Quellenweg 10**
**CH-4402 Frenkendorf (CH)**
Erfinder : **Kerb, Ulrich, Dr.**
**Prinzregentenstrasse 7**
**D-1000 Berlin (DE)**
Erfinder : **Wiechert, Rudolf, Prof.**
**Petzower Strasse 8a**
**D-1000 Berlin (DE)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 052 799 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue D-Homosteroide der Formel

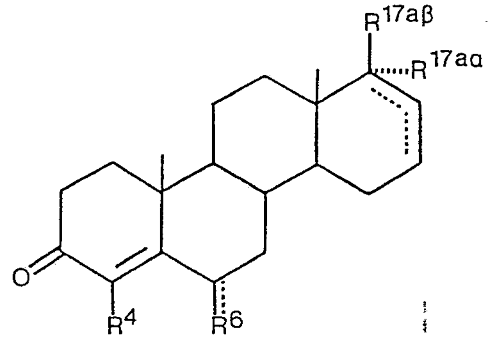

I

worin $R^4$ Wasserstoff, Methyl, Chlor oder Hydroxy ; $R^6$ Wasserstoff oder Methylen ; $R^{17a\alpha}$ Wasserstoff, Methyl oder Chlor und $R^{17a\beta}$ Carboxy, verestertes Carboxy, Cyano, Formyl, Carbamoyl, mono- oder dinieder-Alkyl-Carbamoyl ; oder $R^{17a\alpha}$ und $R^{17a\beta}$ zusammen einen Spiroätherring der Formel

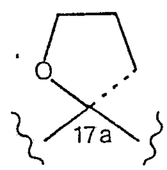

bedeuten und worin die punktierten Bindungen im D-Ring eine fakultative 16(17)- oder 17(17a)-Doppelbindung darstellen, wobei im letzteren Falle $R^{17a\alpha}$ abwesend ist.

Die Erfindung betrifft weiterhin ein Verfahren zur Herstellung der D-Homosteroide der Formel I sowie D-Homosteroide enthaltende pharmazeutische Präparate.

Unter « nieder »-Alkyl allein oder in Kombination werden geradkettige oder verzweigte Alkylgruppen mit 1-7, vorzugsweise 1-4 C-Atomen verstanden.

Der Ausdruck « verestertes Carboxy » bezieht sich auf Carboxylgruppen, die mit einem aliphatischen, cycloaliphatischen, aromatischen oder araliphatischen Rest verestert sind. Beispiele von veresterten Carboxygruppen sind Alkoxycarbonyl, insbesondere ggf. durch Halogen, wie Chlor, oder Amino substituiertes nieder-Alkoxycarbonyl, wie Methoxy-, Aethoxy-, Propoxy- und Butoxycarbonyl ; Cycloalkoxycarbonyl, wie Cyclohexyloxy- und Cyclopentyloxycarbonyl ; Aryloxycarbonyl, wie Phenyloxy- und Tolyloxycarbonyl ; und Aralkoxycarbonyl, wie Benzyloxycarbonyl. Beispiele von mono-nieder-Alkylcarbamoylgruppen sind Methyl-, Aethyl- und Propyl-carbamoyl ; und nieder-Alkyl-amino-substituiertes nieder-Alkylcarbamoyl, wie Di-nieder-alkylamino-äthyl-carbamoyl. Beispiele von di-nieder-Alkylcarbamoylgruppen sind Dimethyl-, Diäthyl-, Dipropyl- und N-Methyl-N-Aethyl-carbamoyl.

Bevorzugte D-Homosteroide der Formel I sind solche, in denen $R^4$, $R^6$ und $R^{17a\alpha}$ Wasserstoff und $R^{17a\beta}$ Carboxy, nieder-Alkoxycarbonyl oder Cyano, oder $R^{17a\alpha}$ und $R^{17a\beta}$ zusammen eine wie oben definierten Spiroätherring darstellen. Besonders bevorzugt sind davon die Verbindungen mit $R^{17a\beta}$ = Carboxy oder nieder-Alkoxycarbonyl und gesättigtem D-Ring, wie die 3-Oxo-D-homo-androst-4-en-17aβ-carbonsäure und deren Methylester.

Die D-Homosteroide der Formel I können erfindungsgemäss dadurch hergestellt werden, dass man

a) ein D-Homosteroid der Formel

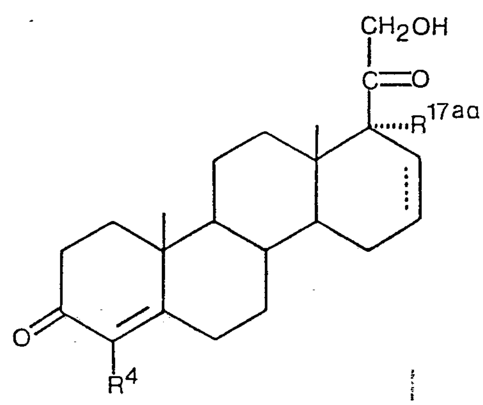

II

mit einem Oxidationsmittel behandelt ; oder

b) ein D-Homosteroid der Formel

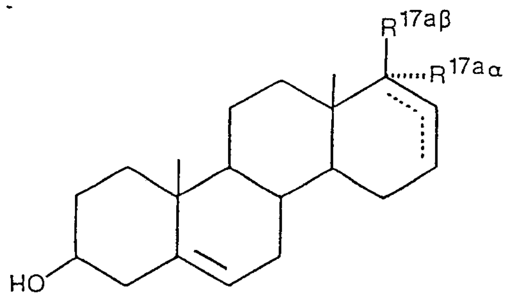

III

mit einem Oxidationsmittel behandelt ; oder

c) ein D-Homosteroid der Formel

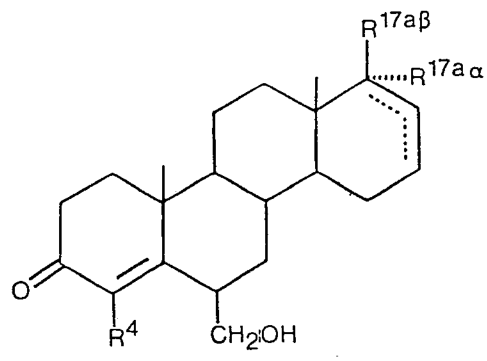

IV

dehydratisiert ; oder

d) ein D-Homosteroid der Formel

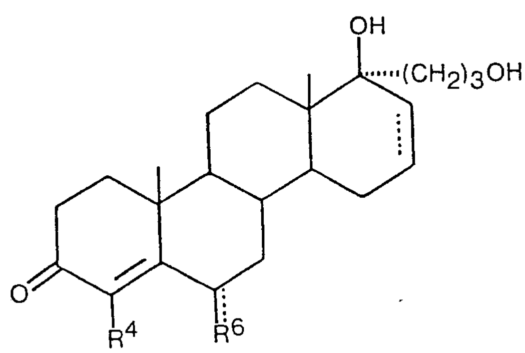

V

zum Aether cyclisiert ; oder

e) in einem D-Homosteroid der Formel I, in der $R^{17a\beta}$ Carboxy ist, die Carboxygruppe verestert ; oder

f) in einem D-Homosteroid der Formel I, in der $R^{17a\beta}$ verestertes Carboxy ist, die veresterte Carboxygruppe verseift ; oder

g) ein D-Homosteroid der Formel I, in der $R^4$ und $R^6$ Wasserstoff sind, in 4-Stellung methyliert, chloriert oder hydroxyliert ; oder

h) in einem D-Homosteroid der Formel I, in der $R^{17a\beta}$ Carboxy ist, die Carboxygruppe amidiert ; oder

i) in einem D-Homosteroid der Formel I, in dem $R^{17a\beta}$ Carbomoyl ist, die Carbamoylgruppe in die Cyanogruppe umwandelt.

Die Oxidation eines D-Homosteroids der Formel II gemäss Verfahrensvariante a) liefert ein D-Homosteroid der Formel I, worin $R^{17a\beta}$ Carboxy ist. Als Oxidationsmittel kommen Reagentien wie Perjodsäure in Betracht. Die Oxidation wird zweckmässig bei Raumtemperatur durchgeführt. Als Lösungsmittel eignen sich solche, in denen beide Reaktionspartner löslich sind, z. B. wässrige Alkohole.

Die Oxidation der 3-Hydroxy-$\Delta^5$-Gruppierung eines D-Homosteroids der Formel III zu entsprechenden 3-Keto-$\Delta^4$-D-Homosteroiden (Verfahrensvariante b) kann mittels der Oppenauer-Reaktion, z. B. mit Aluminiumisopropylat oder mit Oxidationsmitteln wie Chromtrioxid (z. B. Jones' Reagens) oder nach Pfitzner-Moffatt mit Dimethylsulfoxid/Dicyclohexylcarbodiimid (wobei das primär erhaltene $\Delta^5$-3-Keton anschliessend zum $\Delta^4$-3-Keton isomerisiert werden muss), oder mittels Pyridin/$SO_3$ durchgeführt werden.

Die Dehydratisierung der Hydroxymethylgruppe eines D-Homosteroids der Formel IV zur Herstellung eines D-Homosteroids der Formel I in der $R^6$ Methylen ist (Verfahrensvariante c) kann durch Behandlung mit Säuren, wie p-Toluolsulfonsäure, bewerkstelligt werden.

Die Cyclisierungsreaktion gemäss Verfahrensvariante d), die D-Homosteroide der Formel I liefert, in der $R^{17a\alpha}$ und $R^{17a\beta}$ zusammen eine Spiroäthergruppe der Formel

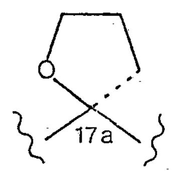

bilden, kann durch Behandlung mit einem Dehydratisierungsmittel, z. B. p-Toluolsulfochlorid oder Methansulfonylchlorid in Pyridin, durchgeführt werden.

Die Veresterung einer 17aβ-Carboxygruppe in einem D-Homosteroid der Formel I (Verfahrensvariante e) kann z. B. durch Behandlung der Säure mit einem Diazoalkan, wie Diazomethan in Aether, oder mit einem O-Alkyl-N,N'-dicyclohexylisoharnstoff in einem aprotischen Lösungsmittel, z. B. Dimethylacetamid oder Dimethylformamid ; oder durch Umsetzung eines Salzes der Säure, z. B. eines Alkalisalzes, mit einem Alkyl-, Cycloalkyl-, Aryl- oder Aralkylhalogenid durchgeführt werden.

Eine veresterte Carboxygruppe $R^{17a\beta}$ kann durch Behandlung mit alkalischen Reagentien, wie alkoholischen Alkalihydroxiden, z. B. methanolischer Kalilauge bei erhöhter Temperatur zur Carboxygruppe verseift werden (Verfahrensvariante f).

Die Chlorierung gemäss Verfahrensvariante g) wird zweckmässigerweise durch Einleiten von Chlor oder Sulfurylchlorid in ein Reaktionsmedium, das das Ausgangssteroid der Formel I enthält, bewerkstelligt. Die Chlorierung kann in Gegenwart eines Protonenakzeptors, beispielsweise Pyridin, Picolin, Dimethylformamid oder Dimethylazetamid ; oder Aethylenoxid oder Propylenoxid, gegebenenfalls unter Zusatz eines weiteren Lösungsmittels, wie Benzol, durchgeführt werden. Andererseits kann die Chlorierung in Abwesenheit eines Protonenakzeptors durchgeführt werden. In diesem Falle wird das primäre Chlorierungsprodukt anschliessend mit einem der genannten Protonenakzeptoren behandelt.

Eine bevorzugte Methode zur Einführung einer 4-Hydroxygruppe besteht in der Umsetzung eines 4,5-gesättigten Ausgangssteroids mit einer starken Base wie Kalium-tert.-butylat in tert.-Butanol unter Belüftung (Sauerstoffzufuhr) (vgl. Tetrahydron Lett. 1961, 554). 4-Hydroxy-D-homosteroide der Formel I können ferner durch alkalische Hydrolyse entsprechender 4-Halogen-D-homosteroide, [vgl. Il Farmaco-Ed. Pr. *17*, 721 (1962)], erhalten werden.

Die 4-Methylierung gemäss Verfahrensvariante g) kann z. B. dadurch vorgenommen werden, dass man ein 4-unsubstituiertes D-Homosteroid der Formel I durch Umsetzung mit Pyrrolidin oder einem anderen sekundären cyclischen Amin, wie Morpholin oder Piperidin, in ein 3-Enamin überführt und dieses mit einem Methylierungsmittel, z. B. einem Methylhalogenid, umsetzt und aus dem Reaktionsprodukt die Enamingruppe durch Hydrolyse abspaltet.

Die Methylierung des 3-Enamins erfolgt bevorzugt in Gegenwart eines organischen Lösungsmittels, wie Aethanol, Methanol, Aethylacetat, insbesondere auch Dimethylformamid etc. Als Halogenid verwendet man zweckmässig das Jodid oder Bromid. Die Hydrolyse der 3-Enamingruppierung zwecks Wiedereinführung der ursprünglichen 3-Keto-$\Delta^4$-gruppierung kann auf an sich bekannte Art erfolgen, z. B. mit Wasser, wässeriger Säure oder Base (beispielsweise mit einer Lösung von Natriumhydroxyd in wässrigem Methanol) oder auch direkt im Verlauf der Alkylierung bzw. Aralkylierung vonstatten gehen, wie z. B. bei Verwendung von Dimethylformamid als Lösungsmittel. Eine weitere Methode zur 4-Methylierung besteht darin, dass man das 3-Keto-$\Delta^4$-Ausgangssteroid mit Formaldehyd und einem Thiol (z. B. einem aliphatischen, aromatischen, alicyclischen oder heterocyclischen Thiol, wie Aethylmercaptan, Thiophenol, Cyclohexylmercaptan, Pyridylmercaptan) in Gegenwart einer Base, insbesondere einer organischen Base, wie eines tertiären Amins, z. B. Trialkylamin, in einen Thiomethyläther, beispielsweise den Phenylthiomethyläther der Formel überführt und letzteren zur $\Delta^4$-4-Methylverbindung reduktiv entschwefelt, z. B. mit desaktiviertem Raney-Nickel (vgl. z. B. Chem. Soc. 1962, 1091).

Weiterhin kann ein 3-Keto-$\Delta^4$-Ausgangssteroid mit einem Methylierungsmittel, insbesondere einem Methyljodid oder -bromid in Gegenwart eines Protonenakzeptors, wie Kalium-tert.-butylat in tert.-Butanol, in 4-Stellung methyliert werden [vgl. z. B. J. Am. Chem. Soc. *85*, 196 (1963)].

Die Umwandlung einer Carboxylgruppe $R^{17a\beta}$ in eine Carbamoylgruppe kann durch Ueberführung der Carbonsäure in ein Säurehalogenid, z. B. mittels eines Acylhalogenids wie Oxalylchlorid, und

Reaktion des Säurehalogenids, mit Ammoniak oder einem mono- oder di-nieder-Alkylamin durchgeführt werden (Verfahrensvariante h).

Die Dehydratisierung eines Säureamids, d. h. eines D-Homosteroids der Formel I, worin $R^{17a\beta}$ Carbamoyl ist, zu einem D-Homosteroid der Formel I, worin $R^{17a\beta}$ Cyano ist, kann mit milden dehydratisierenden Agentien wie Triphenylphosphinoxid/Trifluoracetanhydrid, erreicht werden.

Die als Ausgangsstoffe verwendeten D-Homosteroide der Formeln II-V sind bekannt oder können wie in den nachstehenden Beispielen beschrieben, oder in Analogie dazu, hergestellt werden.

Die D-Homosteroide der Formel I sind therapeutisch wirksam. Insbesondere wirken sie als 5α-Reduktasehemmer und können infolgedessen als anti-Androgene Verwendung finden, beispielsweise zur Behandlung von Akne, Hirsutismus und Prostatahypertrophie. Die D-Homosteroide der Formel I können topisch (z. B. bei Akne), enteral oder parenteral verabreicht werden.

Aus der EP-A-0 013 959 und der FR-A-2 305 976 sind D-Homosteroide mit einer 17α-Carbonsäurefunktion bekannt. Diese D-Homosteroide wirken anti-inflammatorisch. Aus der US-A-3 917 829 sind normale Steroid-17-carbonsäuren mit anti-androgener Wirkung bekannt. Anderer normale Steroide (d. h. Steroide mit 5-gliedrigem D-Ring) mit anti-androgener Wirkung sind aus der US-A-3 705 179 bekannt. Aus der US-A-3 254 074 sind 17-Spiroäther-steroide der Normalreihe bekannt, die die Wirkung von Aldosteron hemmen.

Die Aktivität der D-Homosteroide der Formel I bei der Hemmung der 5α-Reduktase in vitro wurde nach einer modifizierten Methode von M. S. P. Manandhar und J. A. Thomas (Investigat. Urology *14* (1976) 20-22) bestimmt. Als Enzympräparat diente ein 10 %iges (Gewicht/Volumen) Homogenat von ventralen Prostatadrüsen von Albinoratten (180-200 g Gewicht) in eiskaltem 0,05 M Glycylglycinpuffer (pH 7,0). Das Homogenat wurde durch Nylongaze filtriert und vor der Durchführung des Tests mit Pufferlösung auf 1 % verdünnt.

Als Substrat dienten 6 Picomole [1,2,6,7-$^3$H] Testosteron (83 Ci/nMol) in 100 μm̊ Benzol, das in einem Stickstoffstrom abgedampft wurde. Dazu wurden 1,5 ml eines NADPH-liefernden Systems bestehend aus $10^{-4}$ M NADP, 5 I. U. Glucose-6-phosphatdehydrogenase, $4 \times 10^{-3}$ M Glucose-6-phosphat und $5 \times 10^{-5}$ M Dithiothreit gegeben und das Gemisch wurde 10 Minuten bei 37 °C im Wasserbad unter Schütteln präinkubiert. Die Testverbindungen wurden in 20 μl Dimethylsulfoxid gelöst zugegeben, wobei ein Konzentrationsbereich eingehalten wurde, der das 0,1 bis 50-fache der Substratkonzentration vor der prä-Inkubation betrug. Von allen Konzentrationen wurden Dreifachbestimmungen vorgenommen. Die Reaktion wurde durch Zusatz von 0,5 ml Homogenat eingeleitet und die Inkubation nach 6 Minuten durch Zusatz von 5 ml Dichlormethan beendet. Nach Extraktion mit 15 ml Dichlormethan und Zusatz von nichtmarkierten 3α-Androstandiol, 5α-Androstandion, Androstendion, Epi-androsteron, Testosteron und 5α-Dihydrotestosteron (je 20 μg) wurden die Extrakte zur Trockene eingedampft und der Rückstand in einem Minimum von Methanol gelöst und einer Dünnschichtchromatographie auf Silicagel 60 (E. Merck) mit Benzol Aceton (85 : 15) als Lösungsmittel unterworfen. Die aufgetrennten Steroide wurden durch Besprühen mit konzentrierter Schwefelsäure : Aethanol (1 : 1) und 10 Minuten Heizen auf 100 °C sichtbar gemacht. Die entsprechenden Flecke wurden ausgschnitten, in Zählröhrchen verbracht und in einem Scintillationszähler gemessen.

Die in vivo-Prüfung wurde wie folgt durchgeführt : Erwachsene männliche Albinoratten im Gewicht von 200 g wurden kastriert. 2 Stunden später erhielten sie die erste von 10 täglichen Dosen der Testsubstanz, suspendiert in 0,5 % Carboxymethylcellulose, 0,4 % Tween 80, 0,9 % Benzylalkohol in physiologischer Kochsalzlösung. 3 Stunden nach der letzten Medikation am zehnten Behandlungstag wurden die Tiere dekapitiert, das Blut aus dem Rumpf wurde gesammelt und die Samenblasen, die ventrale Prostata und der Muskulus levator ani wurden entfernt und gewogen. Die Resultate sind als ng NIAMDD-RP-I/ml angegeben.

Versuchssubstanzen :

A 3-Oxo-D-homo-androst-4-en-17aβ-carbonsäure
B 3-Oxo-D-homo-androst-4,17-dien-17a-carbonsäure-(2-diäthylamino)-äthylester
C 17a-Methyl-3-oxo-D-homo-androsta-4,16-dien-17aβ-carbonsäuremethylester
D 4-Chlor-3-oxo-D-homo-androst-4-en-17aβ-carbonsäuremethylester
E 3-Oxo-D-homo-androsta-4,17-dien-17a-carbonsäure-methylamid
F 4,5-Dihydrospiro[furan-2(3H), 17'a(β1)-D-homo-androsta-4,16-dien]-3'-on
G 3-Oxo-D-homo-androst-4-en-17aβ-carbonsäuremethylester
H 3-Oxo-D-homo-androsta-4,17-dien-17a-carbonsäurechlormethylester
I 3-Oxo-4-methyl-D-homo-androst-4-en-17aβ-carbonsäuremethylester
J 3-Oxo-D-homo-androst-4-en-17aβ-carbonsäureamid
K 4,5-Dihydro-6'-methylenspiro[furan-2(3H), 17'a(β1)-D-homo-androsta-4,16-dien]-3'-on
L 3-Oxo-D-Homo-androsta-4,17-dien-17a-carbonsäuremethylester
M 3-Oxo-D-homo-androst-4-en-17aβ-carbonsäure-dimethylamid
N 3-Oxo-D-homo-androst-4-en-17aβ-carbonsäurenitril

Resultate :

a) Hemmung der 5α-Reduktase in vitro

| Versuchssubstanz | $IC_{50}$ |
|---|---|
| A | 1 : 4 |
| B | 1 : 2 |
| C | 1 : 6 |
| D | 1 : 2 |
| E | 1 : 5 |
| F | 1 : 6 |
| G | 1 : 0,3 |
| H | 1 : 4 |
| I | 1 : 16 |
| J | 1 : 7 |
| K | 1 : 1 |
| L | 1 : 2 |
| M | 1 : 4 |
| N | 1 : 0,8 |

$IC_{50}$ bezeichnet das molare Verhältnis von Testosteron : Versuchssubstanz, bei dem die Umwandlung des Testosterons in Dihydrotestosteron auf die Hälfte reduziert ist.

b) Hemmung der 5α-Reduktase in vivo

| Versuchs-Substanz | Dosis (mg/kg) | VP (mg) | SV (mg) | MLA (mg) | LH (ng/ml) | FSH (ng/ml) |
|---|---|---|---|---|---|---|
| Kontrolle sc | - | 20 ± 1 | 50 ± 3 | 61 ± 3 | 325 ± 20 | 1040 ± 51 |
| Testosteron-propionat (TP) | 0,5 | 160 ± 18 | 215 ± 13 | 137 ± 7 | 101 ± 36 | 783 ± 167 |
| A sc + TP sc | 15 + 0,5 | 116 ± 11 | 157 ± 15 | 118 ± 8 | 283 ± 41 | 1268 ± 125 |
| Kontrolle sc | - | 48 ± 4 | 38 ± 6 | 60 ± 10 | 271 ± 18 | 1055 ± 35 |
| TP sc | 0,5 | 108 ± 6 | 69 ± 3 | 75 ± 4 | 121 + 12 | 915 ± 37 |
| L sc + TP sc | 15 + 0,5 | 80 ± 6 | 52 ± 1 | 67 ± 6 | 163 ± 30 | 1099 ± 94 |
| Kontrolle sc | - | 16 ± 1 | 20 ± 1 | 52 ± 2 | 402 ± 18 | 1289 ± 50 |
| Testosteron sc (T) | 0,5 | 112 ± 10 | 65 ± 5 | 108 ± 5 | 104 ± 25 | 1206 ± 114 |
| D sc + T sc | 10 + 0,5 | 95 ± 10 | 51 ± 9 | 83 ± 6 | 244 ± 45 | 1325 ± 93 |

(Fortsetzung)

| Versuchs-Substanz | Dosis (mg/kg) | VP (mg) | SV (mg) | MLA (mg) | LH (ng/ml) | FSH (ng/ml) |
|---|---|---|---|---|---|---|
| Kontrolle po | - | 16 ± 1 | 24 ± 2 | 47 ± 4 | - | - |
| T sc | 0,5 | 96 ± 10 | 92 ± 13 | 109 ± 8 | - | - |
| L po | 30 | | | | | |
| + | + | 81 ± 16 | 68 ± 13 | 88 ± 17 | - | - |
| T sc | 0,5 | | | | | |
| Kontrolle sc | - | 16 ± 1 | 18 ± 2 | 51 ± 2 | 283 ± 27 | - |
| T sc | 0,5 | 128 ± 12 | 85 ± 7 | 126 ± 7 | 96 ± 37 | - |
| F sc | 10 | | | | | |
| + | + | 93 ± 10 | 66 ± 4 | 105 ± 6 | 88 ± 15 | - |
| T sc | 0,5 | | | | | |

VP : Ventrale Prostata, SV : Samenblase, MLA : Musculus levator ani, LH : Luteinisierendes Hormon, FSH : Follikel stimulierendes Hormon

Die D-Homosteroide der Formel I können als Heilmittel z. B. in Form pharmazeutischer Präparate Verwendung finden, welche sie oder ihre Salze in Mischung mit einem für die enterale, perkutane oder parenterale Applikation geeigneten pharmazeutischen, organischen oder anorganischen inerten Trägermaterial, wie z. B. Wasser, Gelatine, Gummi arabicum, Milchzucker, Stärke, Magnesiumsulfat, Talk, pflanzliche Oele, Polyalkylenglykole, Vaseline, usw. enthalten. Die pharmazeutischen Präparate können in fester Form, z. B. als Tabletten, Dragées, Suppositorien, Kapseln ; in halbfester Form, z. B. als Salben ; oder in flüssiger Form, z. B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten.

## Beispiel 1

10,0 g 21-Hydroxy-D-homo-pregn-4-en-3,20-dion wurden in 500 ml Methanol gelöst und mit einer Lösung von 15,0 g Perjodsäure in 100 ml Wasser versetzt. Die Reaktionslösung wurde 2 1/2 Stunden bei Raumtemperatur gehalten. Zur Aufarbeitung wurde auf 3 l Wasser gegossen, der ausgefallene Niederschlag abgenutscht und im Vakuum über KOH bei 60° getrocknet. Der Rückstand wurde aus Methylenchlorid-Isopropyläther umkristallisiert. Die reine 3-Oxo-D-homo-androst-4-en-17aβ-carbonsäure schmolz bei 238-240°. $[\alpha]_D^{25} = +98°$ (c = 0,1 in Dioxan).

## Beispiel 2

Eine Mischung von 3,8 g 3-Oxo-D-homo-androst-4-en-17aβ-carbonsäure, 1,90 ml Methyljodid, 1,90 g Natriumhydrogencarbonat und 20 ml N,N-Dimethylacetamid wurde 24 Stunden bei Raumtemperatur gerührt. Zur Aufarbeitung wurde auf Eiswasser gegossen, mit Aether extrahiert, die Aether-Extrakte mit $Na_2SO_4$ getrocknet und im Vakuum eingedampft. Der Rückstand wurde auf der 50-fachen Menge Silicagel mit Methylenchlorid-Aceton 99 : 1 chromatographiert. Die dünnschichtchromatographisch einheitlichen Fraktionen wurden aus Aether-Hexan umkristallisiert und lieferten reinen 3-Oxo-D-homo-androst-4-en-17aβ-carbonsäure-methylester, Smp. 132-133°, $[\alpha]_D^{25} = +147°$ (c = 0,1 in Dioxan).

Analog Beispiel 2 wurden hergestellt :

aus 3-Oxo-D-homo-androsta-4,17-dien-17a-carbonsäure und Isopropyljodid

der 3-Oxo-D-homo-androsta-4,17-dien-17a-carbonsäure-isopropylester, Smp. 83-84°, $[\alpha]_D^{25} = +180°$ (c = 1,0 in Dioxan) ;

aus 3-Oxo-D-homo-androsta-4,17-dien-17a-carbonsäure und Diäthylaminoaethanol
der 3-Oxo-D-homo-androsta-4,17-dien-17a-carbonsäure-(2'-diäthylamino)äthylester ; Smp. 75-77° (Aceton-Hexan) ; $[\alpha]_D^{20} = 158°$ (c = 1,0 in Dioxan),

aus 3-Oxo-D-homo-androsta-4,17-dien-17a-carbonsäure und Chlorjodmethan
der 3-Oxo-D-homo-androsta-4,17-dien-17a-carbonsäure-chlormethylester, Smp. 144-145° (Aceton-Hexan) ; $[\alpha]_D^{20} = +179°$ (c = 0,1 in Dioxan) ;

## Beispiel 3

Eine Mischung von 13,8 g 3β-Hydroxy-D-homo-androsta-5,17-dien-17aβ-carbonsäure-methylester, 600 ml abs. Toluol, 270 ml Cyclohexanon und 16,6 g Aluminium-tert. butylat wurde 1 1/2 Stunden unter Rückfluss am Wasserabscheider erhitzt. Die Reaktionsmischung wurde abgekühlt, auf Eiswasser gegossen und mit Methylenchlorid extrahiert. Der Extrakt wurde mit Wasser gewaschen, mit $Na_2SO_4$ getrocknet und im Vakuum eingedampft. Den Rückstand chromatographierte man auf 1 kg Silicagel. Mit Methylenchlorid-Aceton 98 : 2 wurde reiner 3-Oxo-D-homo-androsta-4,17-dien-17a-carbonsäure-methylester vom Schmelzpunkt 140-141° eluiert. $[\alpha]_D^{25} = +178°$ (c = 0,1 in Dioxan).

Das Ausgangsmaterial wurde wie folgt hergestellt :

3β-Acetoxy-D-homo-androst-5-en-17a-on wurde mit Dichlormethyllithium bei − 60° umgesetzt und das intermediär entstehende « Dichlorepoxyd » durch Erhitzen in Toluol in 3β-Acetoxy-17aα-chlor-D-homo-androst-5-en-17a-carboxyaldehyd vom Schmelzpunkf 149-151° umgewandelt. Der Aldehyd wurde mit Chromsäure zu 3β-Acetoxy-17aβ-chlor-D-homo-androst-5-en-17a-carbonsäure oxidiert. Behandlung der Carbonsäure mit $Li_2CO_3$/DMF bei 60° lieferte 3β-Acetoxy-D-homo-androsta-5,17-dien-17a-carbonsäure, aus der mit Methyljodid in Dimethylacetamid in Gegenwart von $NaHCO_3$ der Methylester, Smp. 182-183°, erhalten wurde. Verseifung des Methylesters mit Natriummethylat in Methanol bei Ramtemperatur führte zum 3β-Hydroxy-D-homo-androsta-5,17-dien-17aβ-carbonsäure-methylester, Smp. 197-198°.

## Beispiel 4

Analog Beispiel 3 wurde hergestellt :

aus 3β-Hydroxy-17a-methyl-D-homo-androsta-5,16-dien-17aβ-carbonsäure-methylester
der 17a-Methyl-3-oxo-D-homo-androsta-4,16-dien-17aβ-carbonsäure-methylester, Smp. 130-132°, $[\alpha]_D^{25} = +22,6°$ (c = 0,7 in Dioxan).

Das Ausgangsmaterial wurde hergestellt durch Reaktion von 3β-Hydroxy-D-homo-androsta-5,17-dien-17aβ-carbonsäure-methylester mit Methyljodid in DMF in Gegenwart von Kalium-tert.butylat bei 50°.

## Beispiel 5

Analog Beispiel 3 wurde hergestellt :

aus 3β-Hydroxy-17a-methyl-D-homo-androst-5-en-17aβ-carbonsäure-methylester
der 17a-Methyl-3-oxo-D-homo-androst-4-en-17aβ-carbonsäure-methylester, Smp. 123-124° ; $[\alpha]_D^{25} = +84°$ (c = 1,0 in Dioxan).

Das Ausgangsmaterial wurde durch kat. Hydrierung von 3β-Hydroxy-17a-methyl-D-homo-androsta-5,16-dien-17aβ-carbonsäure-methylester erhalten.

## Beispiel 6

Analog Beispiel 3 wurde hergestellt :

aus 3β-Hydroxy-D-homo-androsta-5,17-dien-17a-carboxaldehyd
der 3-Oxo-D-homo-androsta-4,17-dien-17a-carboxaldehyd, Smp. 145-148° ; $[\alpha]_D^{25} = +178°$ (c = 0,1 in Dioxan).

Das Augsgangsmaterial wurde aus 3β-Acetoxy-17aα-chlor-D-homo-androst-5-en-17a-carboxaldehyd durch Abspaltung des Chlors mit Lithiumcarbonat in DMF und anschliessende Verseifung des 3-Acetats mit Kaliumcarbonat in Methanol erhalten.

Beispiel 7

Eine Mischung von 4,0 g 3-Oxo-D-homo-androsta-4,17-dien-17a-carbonsäure-methylester, 10 g Lithiumjodid und 300 ml Collidin wurde 16 Stunden unter Rückfluss erhitzt. Zur Aufarbeitung wurde auf Eiswasser gegossen, mit KOH auf pH 11 eingestellt und dreimal mit Aether extrahiert. Die wässerigalkalische Lösung wurde mit Salzsäure auf pH 2 angesäuert und dreimal mit Aether-Methylenchlorid 3 : 1 extrahiert. Der Aether-Methylenchlorid-Extrakt wurde mit Wasser gewaschen, mit Natriumsulfat getrocknet und im Vakuum eingedampft. Den Rückstand kristallisierte man aus Methanol und erhielt reine 3-Oxo-D-homo-androsta-4,17-dien-17a-carbonsäure, Smp. 229-231° ; $[\alpha]_D^{25} = + 205°$ (c = 0,1 in Dioxan).

Nach der gleichen Methode wurde aus 4-Methyl-3-oxo-D-homo-androsta-4,17-dien-17a-carbonsäure-methylester die 4-Methyl-3-oxo-D-homo-androsta-4,17-dien-17a-carbonsäure ; Smp. 280-283 ; $[\alpha]_D^{25} = + 210°$ (c = 0,5 in Dioxan) ; und aus 4-Methyl-3-oxo-D-homo-androst-4-en-17aβ-carbonsäure-methylester die 4-Methyl-3-oxo-D-homo-androst-4-en-17aβ-carbonsäure erhalten ; Smp. 258-259° ; $[\alpha]_D^{25} = + 147°$ (c = 1,0 in Dioxan).

Beispiel 8

Zu einer gut gerührten, unter Rückfluss siedenden Lösung von 2,0 g 3-Oxo-D-homo-androst-4-en-17aβ-carbonsäure-methylester und 1,16 g Kalium-tert.butylat in 40 ml tert.Butanol tropfte man während 2 1/2 Stunden eine Mischung von 0,55 ml Methyljodid und 100 ml tert.Butanol. Die Reaktionsmischung wurde dann abgekühlt, mit 20 ml 2 N HCl angesäuert, im Vakuum auf ein kleines Volumen eingeengt, auf Wasser gegossen und mit Aether extrahiert. Die Aether-Extrakte wusch man mit Wasser, trocknete mit $Na_2SO_4$ und dampfte im Vakuum ein. Der Rückstand wurde auf 150 g Silicagel chromatographiert. Die mit Hexan-Aether 3 : 1 eluierten, reinen Fraktionen wurden aus Aceton-Hexan umkristallisiert und lieferten reinen 3-Oxo-4-methyl-D-homo-androst-4-en-17aβ-carbonsäure-methylester, Smp. 130-132° ; $[\alpha]_D^{25} = + 147°$ (c = 1,0 in Dioxan).

Beispiel 9

Zu einer Lösung von 0,684 g 3-Oxo-D-homo-androsta-4,17-dien-17a-carbonsäure-methylester in 10 ml Pyridin gab man unter Rühren tropfenweise 0,34 ml Sulfurylchlorid. Die Lösung wurde 15 Minuten bei Raumtemperatur gehalten, dann auf Eiswasser gegossen und mit Methylenchlorid extrahiert. Der Extrakt wurde mit verdünnter Salzsäure und Wasser neutral gewaschen, mit $Na_2SO_4$ getrocknet und im Vakuum eingedampft. Der Rückstand wurde auf 30 g Silicagel chromatographiert. Mit Hexan-Aether 3 : 1 konnte reiner 4-Chlor-3-oxo-D-homo-androsta-4,17-dien-17a-carbonsäure-methylester eluiert werden. Smp. 148-152° (Aceton-Hexan) ; $[\alpha]_D^{25} = + 11,8°$ (c = 1,0 in Dioxan).

Analog wurde hergestellt :

aus 3-Oxo-D-homo-androst-4-en-17aβ-carbonsäure-methylester der 4-Chlor-3-oxo-D-homo-androst-4-en-17aβ-carbonsäure-methylester, Smp. 163-164° ; $[\alpha]_D^{25} = + 154,8°$ (c = 0,8 in Dioxan).

Beispiel 10

Eine Mischung von 2,0 g 3-Oxo-D-homo-androsta-4,17-dien-17a-carbonsäure-methylester, 1,6 g Thiophenol, 2,0 g 40 % Formaldehydlösung und 4,0 g Triäthanolamin wurde 18 Stunden unter Rückfluss erhitzt. Dann wurde auf Eiswasser gegossen, mit Methylenchlorid extrahiert, die Extrakte mit verdünnter Salzsäure, verdünnter Natronlauge und Wasser neutral gewaschen, mit $Na_2SO_4$ getrocknet und im Vakuum eingedampft. Man erhielt 2,7 g rohe 4-Phenyl-thiomethyl-Verbindung, welche in 70 ml Aceton mit 10 g desaktiviertem Raney-Nickel 40 Stunden unter Rückfluss erhitzt wurde. Das abgekühlte Reaktionsgemisch wurde filtriert, das Filtrat im Vakuum eingedampft. Den Rückstand chromatographierte man auf 150 g Silicagel. Mit Methylenchlorid konnte 0,9 g reiner 4-Methyl-3-oxo-D-homo-androsta-4,17-dien-17a-carbonsäure-methylester eluiert werden. Smp. 121-122° ; $[\alpha]_D^{25} = + 212°$ (c = 0,29 in Dioxan).

Beispiel 11

289 mg 17a-Chlor-3β-hydroxy-D-homoandrost-5-en-17aβ-carbonsäure-methylester wurden bei 0° unter Argon in 23 ml Aceton mit 0,285 ml Jones-Reagens versetzt. Nach 5 Minuten wurde mit 1 ml Isopropanol gewaschen und nach weiteren 5 Minuten wurde das Reaktionsgemisch mit Wasser und Methylenchlorid verdünnt. Die organische Phase wurde mit Wasser neutral gewaschen, getrocknet und im Vakuum eingedampft. Der erhaltene Rückstand wurde in 8 ml Eisessig und 0,8 ml 2 N Salzsäure 1 Stunde bei 20° gerührt. Das Reaktionsgemisch wurde zur Trockene eingedampft. Chromatographie des Rückstandes an Kieselgel gab 17a-Chlor-3-oxo-D-homo-androst-4-en-17aβ-carbonsäure-methylester, Smp. 163° ; $[\alpha]_D^{25} = + 53°$ (c = 0,1 % in Dioxan).

Beispiel 12

Eine Mischung von 0,68 g 3-Oxo-D-homo-androsta-4,17-dien-17a-carbonsäure-methylester, 15 ml Methanol, 0,7 ml 10 % NaOH und 1,4 ml 30 % $H_2O_2$ wurde 23 Stunden bei 0° gehalten. Zur Aufarbeitung wurde mit 0,2 ml Essigsäure versetzt, auf Eiswasser gegossen und mit Methylenchlorid extrahiert. Der Extrakt wurde im Vakuum eingedampft, in 30 ml Methanol gelöst und mit 6 ml Wasser und 6 ml 2 N Schwefelsäure versetzt. Die Reaktionsmischung wurde 40 Stunden bei Raumtemperatur gehalten, dann auf Eiswasser gegossen und mit Methylenchlorid extrahiert. Den Extrakt wusch man mit Wasser, trocknete mit $Na_2SO_4$, dampfte im Vakuum ein und chromatographierte den Rückstand auf Silicagel. Mit Methylenchlorid konnte reines 4-Hydroxy-3-oxo-D-homo-androsta-4,17-dien-17a-carbonsäure-methylester eluiert werden. Smp. 176-179° ; $[\alpha]_D^{25} = + 192,6°$ (c = 0,5 in Dioxan).

Analog wurde hergestellt :

aus 3-Oxo-D-homo-androst-4-en-17aβ-carbonsäure-methylester
der 4-Hydroxy-3-oxo-D-homoandrost-4-en-17aβ-carbonsäure-methylester, Smp. 184-187° ; $[\alpha]_D^{25} = + 127°$ (c = 0,73 in Dioxan).

Beispiel 13

Zu einer auf 0° abgekühlten Lösung von 0,713 g 3β-Hydroxy-D-homo-androst-5-en-17β-carboxaldehyd in 20 ml Aceton und 20 ml Methylenchlorid tropfte man unter Rühren eine Mischung von 0,68 ml Jones-Reagens in 2 ml Aceton. Anschliessend wurde noch 15 Minuten gerührt, mit 1 ml Isopropylalkohol versetzt, auf Eiswasser gegossen und mit Methylenchlorid extrahiert. Uebliche Aufarbeitung lieferte 650 mg Rohprodukt, das in 20 ml Essigsäure und 2 ml 2 N Salzsäure gelöst, 2 Stunden bei Raumtemperatur gehalten wurde. Zur Aufarbeitung wurde auf Eiswasser gegossen, mit Methylenchlorid extrahiert, der Methylenchlorid-Extrakt mit Wasser gewaschen, mit $Na_2SO_4$ getrocknet und im Vakuum eingedampft. Der Rückstand wurde auf Silicagel chromatographiert. Mit Methylenchlorid konnte reiner 3-Oxo-D-homo-androst-4-17aβ-carboxaldehyd eluiert werden. SMP. 123-125° ; $[\alpha]_D^{25} = + 133°$.

Analog wurde hergestellt :

aus 3β-Hydroxy-17a-methyl-D-homo-androsta-5,16-dien-17aβ-carbonsäure
die 17a-Methyl-3-oxo-D-homo-androsta-4,16-dien-17aβ-carbonsäure, Smp. 270-280° ; $[\alpha]_D^{25} = 0°$ (c = 0,2 in Dioxan).

Beispiel 14

Zu einer auf 5° gekühlten Suspension von 0,99 g 3-Oxo-D-homo-androst-4-en-17aβ-carbonsäure in 30 ml Benzol tropfte man unter Rühren 4,5 ml Oxalylchlorid. Die Reaktionslösung wurde 20 Minuten bei Raumtemperatur gerührt und anschliessend im Vakuum eingedampft. Der Rückstand wurde in 25 ml Benzol gelöst, worauf während 30 Minuten Ammoniak in die Lösung eingeleitet wurde. Das ausgefallene Ammoniumchlorid wurde abfiltriert und das Filtrat im Vakuum eingeengt. Der Rückstand wurde auf Silicagel chromatographiert. Mit Methylenchlorid/Aceton 5 : 1 wurde reines 3-Oxo-D-homo-androst-4-en-17aβ-carbonsäureamid eluiert. Smp. 226-227° (Aceton-Hexan), $[\alpha]_D^{25} = + 114°$ (c = 0,4 in Dioxan).

Analog wurden hergestellt :

aus 3-Oxo-D-homo-androst-4-en-17aβ-carbonsäure und Dimethylamin
das 3-Oxo-D-homo-androst-4-en-17aβ-carbonsäure-dimethylamid ; Smp. 153-154° (Aceton-Hexan) ; $[\alpha]_D^{25} = 115°$ (c = 0,4 in dioxan).

aus 3-Oxo-D-homo-androsta-4,17-dien-17a-carbonsäure und Methylamin
das 3-Oxo-D-homo-androsta-4,17-dien-17a-carbonsäure-methylamid ; Smp. 256-257° (Aceton-Hexan), $[\alpha]_D^{20} = + 196°$ (c = 0,4 in Dioxan).

aus 3-Oxo-D-homo-androst-4-en-17aβ-carbonsäure und (2-Diäthylamino)-äthylamin
das 3-Oxo-D-homo-androst-4-en-17aβ-carbonsäure-(2′-diäthylaminoäthyl)amid-hydrochlorid ; amorph ; $[\alpha]_D^{20} = + 59°$ (c = 0,15 in Dioxan).

Beispiel 15

Zu einer Mischung von 2,1 g Triphenylphosphinoxid und 15 ml Methylenchlorid gab man bei 0-5° unter Rühren eine Lösung von 1,2 ml Trifluoressigsäureanghydrid in 15 ml Methylenchlorid. Es wurde 15 Minuten bei 0-5° gerührt, dann 1,4 g 3-Oxo-D-homo-androst-4-en-17aβ-carbonsäureamid in 10 ml Methylenchlorid zugegeben. Die Reaktionsmischung wurde 16 Stunden bei Raumtemperatur gerührt, dann auf Eiswasser gegossen und mit Methylenchlorid extrahiert. Der organische Extrakt wurde mit Wasser neutral gewaschen, mit $Na_2SO_4$ getrocknet und im Vakuum eingedampft. Den Rückstand

chromatographierte man auf Silicagel. Mit Hexan-Aceton 95 : 5 eluierte man reines 3-Oxo-D-homo-androst-4-en-17aβ-carbonsäurenitril ; Smp. 133-135° ; $[\alpha]_D^{20} = + 137°$ (c = 0,15 in Dioxan).

Beispiel 16

Zu einer Lösung von 44,6 g 17aβ-Hydroxy-17a-(3'-hydroxypropyl)-D-homo-androsta-4,16-dien-3-on in 200 ml Pyridin gab man 28 g p-Toluolsulfochlorid und rührte die Mischung 3 Stunden bei Raumtemperatur. Das Reaktionsgemisch wurde auf Eiswasser gegossen, mit Aether extrahiert, die Aether-Extrakte mit 1 N NaOH und anschliessend mit Wasser gewaschen, mit $Na_2SO_4$ getrocknet und in Vakuum eingedampft. Der Rückstand wurde auf 1,2 kg Silicagel chromatographiert. Mit Toluol-Essigester 95 : 5 konnten 30 g reines 4,5-Dihydrospiro[furan-2(3H); 17'a(β1)-D-homoandrosta-4,16-dien]3'-on isoliert werden. Smp. 131-132° (Aceton-Hexan), $[\alpha]_D^{25} = + 8°$ (c = 0,1 in Methanol).

Das Ausgangsmaterial wurde durch Reduktion von 3-Oxo-D-homo-17aα-pregna-4,16-dien-21,17a-carbolacton mit $LiAlH_4$ und Oxidation des erhaltenen 3-ols mit $MnO_2$ hergestellt.

Beispiel 17

5,65 g 1-[4,5-Dihydrospiro]furan-2(3H), 17'a(β1)-D-homoandrosta-3,5,16-trien-3'-yl]pyrrolidin wurden in 350 ml Benzol, 175 ml Methanol und 10 ml 35 %ige Formaldehydlösung gelöst und die Mischung 45 Minuten bei Raumtemperatur gerührt. Zur Aufarbeitung wurde die Reaktionslösung bei 35° in Vakuum eingedampft und der Rückstand auf 400 g Silicagel chromatographiert. Mit Hexan-Aether 5 : 1 wurde 3,80 g reines 4,5-Dihydro-6'β-(hydroxymethyl)-spiro[furan-2(3H), 17'a(β1)-D-homoandrosta-4,16-dien]-3'-on eluiert, das in 250 ml Dioxan gelöst, mit 10 ml 18 %iger Salzsäure innert 10 Minuten versetzt und anschliessend 1 3/4 Stunden bei Raumtemperatur gerührt wurde. Zur Aufarbeitung gab man 18 g Natriumhydrogencarbonat zu, rührte 1 Stunde bei Raumtemperatur, filtrierte den Niederschlag ab und konzentrierte das Filtrat im Vakuum. Man erhielt 3,5 g beige Kristalle, die auf 150 g Silicagel chromatographiert wurden. Mit Hexan-Aceton 9 : 1 konnte reines 4,5-Dihydro-6'-methylenspiro[furan-2(3H),-17'a(β1)-D-homoandrosta-4,16-dien]-3'-on eluiert werden. Smp. 167-169° ; $[\alpha]_D^{25} = + 154,5°$ (c = 0,6 in Dioxan).

Das Ausgangsmaterial wird wie folgt hergestellt :

5,0 g 4,5-Dihydrospiro[furan-2(3H), 17'a(β1)-D-homo-androsta-4,16-dien]-3'-on wurden in 150 ml Methanol gelöst, mit 3 ml Pyrrolidin versetzt und 5 Minuten unter Rückfluss gekocht. Die Reaktionslösung wurde dann auf − 15° abgekühlt. Das ausgefallene Enamin wurde abgenutscht und im Vakuum bei Raumtemperatur getrocknet. Smp. 176-178°.

Beispiel A

Lotion zur topischen Anwendung, z. B. bei Akne :

| | |
|---|---|
| 3-Oxo-D-homo-androst-4-en-17aβ-carbonsäuremethylester | 2 g |
| 2-Phenyläthylalkohol | 15 ml |
| Glycerin | 5 ml |
| Aethanol q. s. | ad 100 ml |

Beispiel B

Gel :

| | |
|---|---|
| 3-Oxo-D-homo-androst-4-en-17aβ-carbonsäure-(2'-diäthylaminoäthyl)amid-hydrochlorid | 5 g |
| Hydroxypropylcellulose | 2,6 g |
| Isopropanol | 30 g |
| Wasser | 0,1 g |
| Na-Aethylendiamintetraacetat | 0,01 g |
| Propylenglykol q. s. | ad 100 g |

Beispiel C

Salbe :

| | |
|---|---|
| 3-Oxo-D-homo-androst-4-en-17aβ-carbonsäure-(2'-diäthylaminoäthyl)amid-hydrochlorid | 5 g |
| Wachs, weiss | 35 g |
| Wollfettalkohole | 5 g |
| Amerchol C | 15 g |
| Isopropylmyristat q. s. | ad 100 g |

**Ansprüche** (für die Vertragsstaaten : BE, CH, DE, FR, GB, IT, LI, LU, NL, SE)

1. D-Homosteroide der Formel

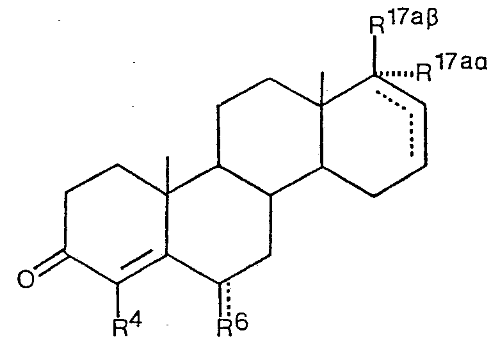

I

worin $R^4$ Wasserstoff, Methyl, Chlor oder Hydroxy ; $R^6$ Wasserstoff oder Methylen ; $R^{17a\alpha}$ Wasserstoff, Methyl oder Chlor und $R^{17a\beta}$ Carboxy, verestertes Carboxy, Cyano, Formyl, Carbamoyl, mono- oder di-nieder-Alkyl-Carbamoyl ; oder $R^{17a\alpha}$ und $R^{17a\beta}$ zusammen einen Spiroätherring der Formel

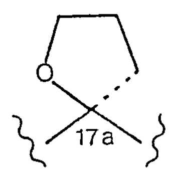

bedeuten und worin die punktierten Bindungen im D-Ring eine fakultative 16(17)- oder 17(17a)-Doppelbindung darstellen.

2. D-Homosteroide gemäss Anspruch 1, worin $R^4$, $R^6$ und $R^{17a\alpha}$ Wasserstoff bedeuten.

3. D-Homosteroide gemäss Anspruch 1, worin $R^{17a\beta}$ Carboxy, nieder-Alkoxycarbonyl oder Cyano oder $R^{17a\alpha}$ und $R^{17a\beta}$ zusammen eine Spiroätherring der Formel

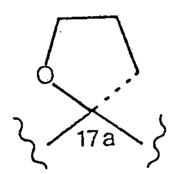

bedeuten.

4. D-Homosteroide gemäss Anspruch 1, worin $R^4$, $R^6$ und $R^{17a\alpha}$ Wasserstoff und $R^{17a\beta}$ Carboxy oder nieder-Alkoxycarbonyl bedeuten und in denen der D-Ring gesättigt ist.

5. 3-Oxo-D-homo-androst-4-en-17aβ-carbonsäure.

6. 3-Oxo-D-homo-androst-4-en-17aβ-carbonsäuremethylester.

7. 3-Oxo-D-homo-androsta-4,17-dien-17a-carbonsäure-isopropylester, 3-Oxo-D-homo-androsta-4,17-dien-17a-carbonsäure-(2'-diäthylamino)äthylester, 3-Oxo-D-homo-androsta-4,17-dien-17a-carbonsäure-chlormethylester, 3-Oxo-D-homo-androsta-4,17-dien-17a-carbonsäure-methylester, 3-Oxo-D-homo-androsta-4,17-dien-17a-carboxaldehyd, 3-Oxo-D-homo-androsta-4,17-dien-17a-carbonsäure, 3-Oxo-D-homo-androst-4-en-17aβ-carboxaldehyd, 3-Oxo-D-homo-androst-4-en-17aβ-carbonsäureamid, 3-Oxo-D-homo-androst-4-en-17aβ-carbonsäure-dimethylamid, 3-Oxo-D-homo-androsta-4,17-dien-17a-carbonsäure-methylamid, 3-Oxo-D-homo-androst-4-en-17aβ-carbonsäure-(2'-diäthylaminoäthyl)amid-hydrochlorid, 3-Oxo-D-homo-androst-4-en-17aβ-carbonsäurenitril.

8. 17a-Methyl-3-oxo-D-homo-androsta-4,16-dien-17aβ-carbonsäure-methylester, 17a-Methyl-3-oxo-D-homo-androst-4-en-17aβ-carbonsäure-methylester, 17a-Chlor-3-oxo-D-homo-androst-4-en-17aβ-carbonsäure-methylester, 17a-Methyl-3-oxo-D-homo-androsta-4,16-dien-17aβ-carbonsäure.

9. 4-Methyl-3-oxo-D-homo-androsta-4,17-dien-17a-carbonsäure, 4-Methyl-3-oxo-D-homo-androst-4-en-17aβ-carbonsäure, 3-Oxo-4-methyl-D-homo-androst-4-en-17aβ-carbonsäure-methylester, 4-Chlor-3-oxo-D-homo-androsta-4,17-dien-17a-carbonsäure-methylester, 4-Chlor-3-oxo-D-homo-androst-4-en-17a-β-carbonsäure-methylester, 4-Methyl-3-oxo-D-homo-androsta-4,17-dien-17a-carbonsäure-methylester, 4-Hydroxy-3-oxo-D-homo-androsta-4,17-dien-17a-carbonsäure-methylester, 4-Hydroxy-3-oxo-D-homo-androst-4-en-17aβ-carbonsäure-methylester.

10. 4,5-Dihydrospiro[furan-2(3H), 17'a(β1)-D-homoandrosta/4,16/dien]-3'-on und 4,5-Dihydro-6'-methylenspiro[furan-2(3H), 17'a(β1)-D-homoandrosta(4,16)dien]-3'-on.

11. D-Homosteroide der Formel I als Heilmittel.

12. D-Homosteroide der Formel I als anti-Androgene.

13. Verfahren zur Herstellung von D-Homosteroiden der Formel I, dadurch gekennzeichnet, dass man

a) ein D-Homosteroid der Formel

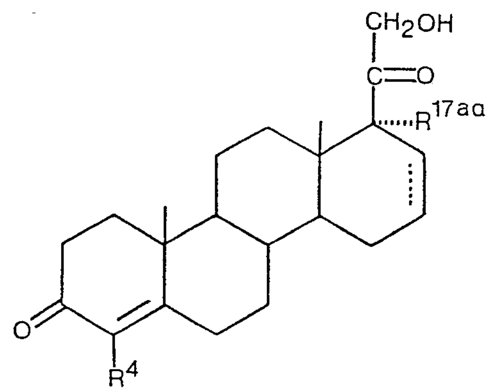

II

mit einem Oxidationsmittel behandelt ; oder

b) ein D-Homosteroid der Formel

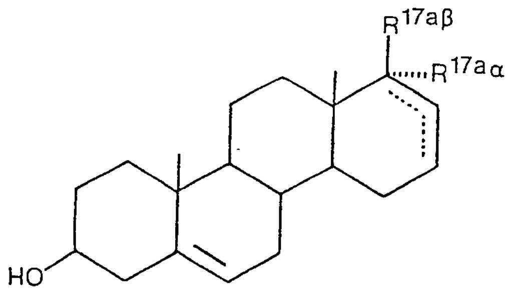

III

mit einem Oxidationsmittel behandelt ; oder

c) ein D-Homosteroid der Formel

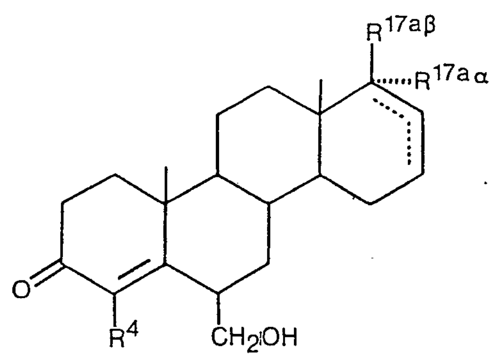

IV

dehydratisiert ; oder

d) ein D-Homosteroid der Formel

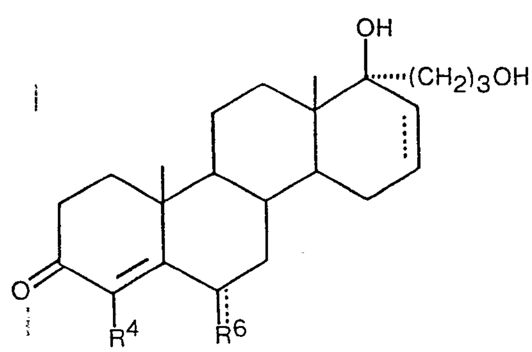

V

zum Aether cyclisiert ; oder

e) in einem D-Homosteroid der Formel I, in der $R^{17a\beta}$ Carboxy ist, die Carboxygruppe verestert ; oder

f) in einem D-Homosteroid der Formel I, in der $R^{17a\beta}$ verestertes Carboxy ist, die veresterte Carboxygruppe verseift ; oder

g) ein D-Homosteroid der Formel I, in der $R^4$ und $R^6$ Wasserstoff sind, in 4-Stellung methyliert, chloriert oder hydroxyliert ; oder

h) in einem D-Homosteroid der Formel I, in der $R^{17a\beta}$ Carboxy ist, die Carboxygruppe amidiert ; oder

i) in einem D-Homosteroid der Formel I, in dem $R^{17a\beta}$ Carbamoyl ist, die Carbamoylgruppe in die Cyanogruppe umwandelt.

14. Pharmazeutische Präparate, enthaltend ein D-Homosteroid der Formel I.

**Ansprüche** (für den Vertragsstaat AT)

1. Verfahren zur Herstellung von D-Homosteroiden der Formel

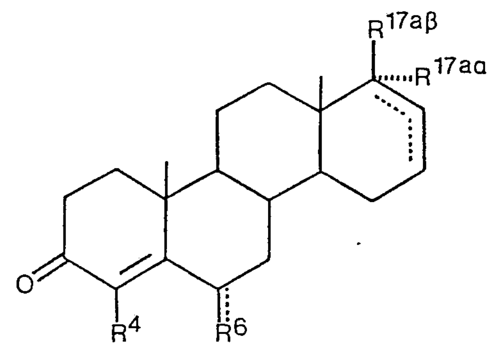

I

worin $R^4$ Wasserstoff, Methyl, Chlor oder Hydroxy ; $R^6$ Wasserstoff oder Methylen ; $R^{17a\alpha}$ Wasserstoff, Methyl oder Chlor und $R^{17a\beta}$ Carboxy, verestertes Carboxy, Cyano, Formyl, Carbamoyl, mono- oder di-nieder-Alkyl-Carbamoyl ; oder $R^{17a\alpha}$ und $R^{17a\beta}$ zusammen einen Spiroätherring der Formel

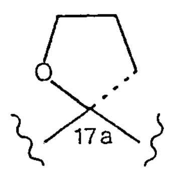

bedeuten und worin die punktierten Bindungen im D-Ring eine fakultative 16(17)- oder 17(17a)-Doppelbindung darstellen,

dadurch gekennzeichnet, dass man

a) ein D-Homosteroid der Formel

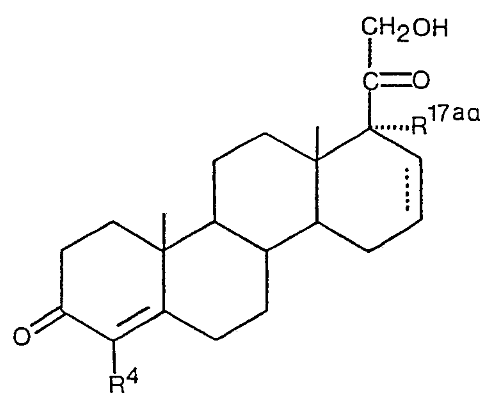

II

mit einem Oxidationsmittel behandelt ; oder

b) ein D-Homosteroid der Formel

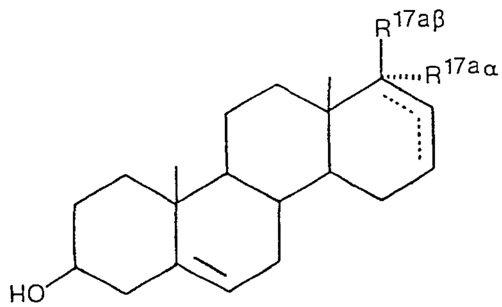

III

mit einem Oxidationsmittel behandelt ; oder

c) ein D-Homosteroid der Formel

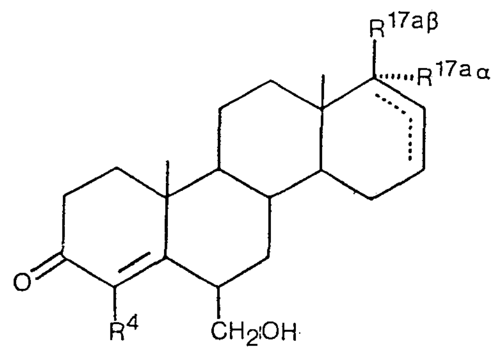

IV

dehydratisiert ; oder

d) ein D-Homosteroid der Formel

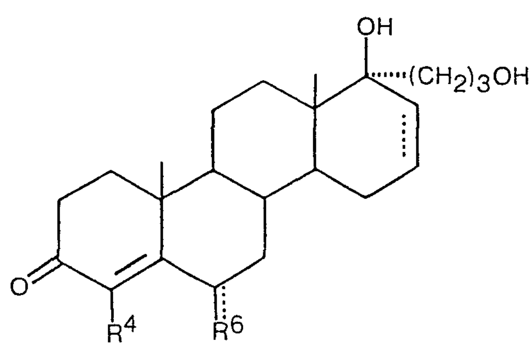

V

zum Aether cyclisiert ; oder

e) in einem D-Homosteroid der Formel I, in der $R^{17a\beta}$ Carboxy ist, die Carboxygruppe verestert ; oder

f) in einem D-Homosteroid der Formel I, in der $R^{17a\beta}$ verestertes Carboxy ist, die veresterte Carboxygruppe verseift ; oder

g) ein D-Homosteroid der Formel I, in der $R^4$ und $R^6$ Wasserstoff sind, in 4-Stellung methyliert, chloriert oder hydroxyliert ; oder

h) in einem D-Homosteroid der Formel I, in der $R^{17a\beta}$ Carboxy ist, die Carboxygruppe amidiert ; oder

i) in einem D-Homosteroid der Formel I, in dem $R^{17a\beta}$ Carbamoyl ist, die Carbamoylgruppe in die Cyanogruppe umwandelt.

2. Verfahren nach Anspruch 1, dadurch gekennzeichnet, dass man D-Homosteroide der Formel I herstellt, worin $R^4$, $R^6$ und $R^{17a\alpha}$ Wasserstoff bedeuten.

3. Verfahren nach Anspruch 1, dadurch gekennzeichnet, dass man D-Homosteroide der Formel I herstellt, worin $R^{17a\beta}$ Carboxy, nieder-Alkoxycarbonyl oder Cyano ; oder $R^{17a\beta}$ zusammen einen Spiroätherring der Formel

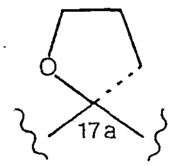

bedeuten.

4. Verfahren nach Anspruch 1, dadurch gekennzeichnet, dass man D-Homosteroide der Formel I herstellt, worin $R^4$, $R^6$ und $R^{17a\alpha}$ Wasserstoff $R^{17a\beta}$ Carboxy oder nieder-Alkoxycarbonyl bedeuten und in denen der D-Ring gesättigt ist.

5. Verfahren nach Anspruch 1, dadurch gekennzeichnet, dass man 3-Oxo-D-homo-androst-4-en-17aβ-carbonsäure herstellt.

6. Verfahren nach Anspruch 1, dadurch gekennzeichnet, dass man 3-Oxo-D-homo-androst-4-en-17aβ-carbonsäuremethylester herstellt.

7. Verfahren nach Anspruch 1, dadurch gekennzeichnet, dass man 3-oxo-D-homo-androsta-4,17-dien-17a-carbonsäure-isopropylester, 3-Oxo-D-homo-androsta-4,17-dien-17a-carbonsäure-(2'-diäthylamino)-äthylester, 3-Oxo-D-homo-androsta-4,17-dien-17a-carbonsäure-chlormethylester, 3-Oxo-D-homo-androsta-4,17-dien-17a-carbonsäure-methylester, 3-Oxo-D-homo-androsta-4,17-dien-17a-carboxaldehyd, 3-Oxo-D-homo-androsta-4,17-dien-17a-carbonsäure, 3-Oxo-D-homo-androst-4-en-17aβ-carboxaldehyd, 3-Oxo-D-homo-androst-4-en-17aβ-carbonsäureamid, 3-Oxo-D-homo-androst-4-en-17aβ-carbonsäure-dimethylamid, 3-Oxo-D-homo-androsta-4,17-dien-17a-carbonsäure-methylamid, 3-Oxo-D-homo-androst-4-en-17aβ-carbonsäure-(2'-diäthylaminoäthyl)amid-hydrochlorid oder 3-Oxo-D-homo-androst-4-en-17aβ-carbonsäurenitril herstellt.

8. Verfahren nach Anspruch 1, dadurch gekennzeichnet, dass man 17a-Methyl-3-oxo-D-homo-androsta-4,16-dien-17aβ-carbonsäure-methylester, 17a-Methyl-3-oxo-D-homo-androst-4-en-17aβ-carbonsäure-methylester, 17a-Chlor-3-oxo-D-homo-androst-4-en-17aβ-carbonsäure-methylester oder 17a-Methyl-3-oxo-D-homo-androsta-4,16-dien-17aβ-carbonsäure herstellt.

9. Verfahren nach Anspruch 1, dadurch gekennzeichnet, dass man 4-Methyl-3-oxo-D-homo-androsta-4,17-dien-17a-carbonsäure, 4-Methyl-3-oxo-D-homo-androst-4-en-17aβ-carbonsäure, 3-Oxo-4-methyl-D-homo-androst-4-en-17aβ-carbonsäure-methylester, 4-Chlor-3-oxo-D-homo-androsta-4,17-dien-17a-carbonsäure-methylester, 4-Chlor-3-oxo-D-homo-androst-4-en-17aβ-carbonsäure-methylester, 4-Methyl-3-oxo-D-homo-androsta-4,17-dien-17a-carbonsäure-methylester, 4-Hydroxy-3-oxo-D-homo-androsta-4,17-dien-17a-carbonsäure-methylester oder 4-Hydroxy-3-oxo-D-homo-androst-4-en-17aβ-carbonsäure-methylester herstellt.

10. Verfahren nach Anspruch 1, dadurch gekennzeichnet, dass man 4,5-Dihydrospiro[furan-2(3H), 17'a(β1)-D-homoandrosta/4,16/dien]-3'-on oder 4,5-Dihydro-6'-methylenspiro[furan-2(3H), 17'a(β1)-D-homoandrosta(4,16)dien]-3'-on herstellt.

**Claims** (for the Contracting States : BE, CH, DE, FR, GB, IT, LI, LU, NL, SE)

1. D-Homosteroids of the formula

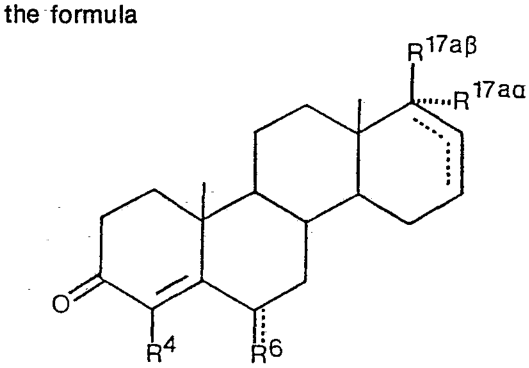

I

wherein $R^4$ signifies hydrogen, methyl, chlorine or hydroxy ; $R^6$ signifies hydrogen or methylene ; $R^{17a\alpha}$ signifies hydrogen, methyl or chlorine and $R^{17a\beta}$ signifies carboxy, esterified carboxy, cyano, formyl, carbamoyl, mono- or di-lower-alkyl-carbamoyl ; or $R^{17a\alpha}$ and $R^{17a\beta}$ together signify a spiroether ring of the formula

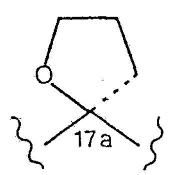

and wherein the dotted bonds in the D-ring represent an optional 16(17)- or 17(17a)-double bond.

2. D-Homosteroids in accordance with claim 1, wherein $R^4$, $R^6$ and $R^{17a\alpha}$ signify hydrogen.

3. D-Homosteroids in accordance with claim 1, wherein $R^{17a\beta}$ signifies carboxy, lower-alkoxycarbonyl or cyano or $R^{17a\alpha}$ and $R^{17a\beta}$ together signify a spiroether ring of the formula

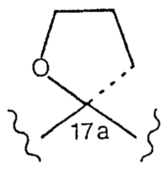

4. D-Homosteroids in accordance with claim 1, wherein $R^4$, $R^6$ and $R^{17a\alpha}$ signify hydrogen and $R^{17a\beta}$ signifies carboxy or lower-alkoxycarbonyl and in which the D-ring is saturated.

5. 3-Oxo-D-homo-androst-4-ene-17aβ-carboxylic acid.

6. Methyl 3-oxo-D-homo-androst-4-ene-17aβ-carboxylate.

7. Isopropyl 3-oxo-D-homo-androsta-4,17-diene-17a-carboxylate, (2′-diethylamino)ethyl 3-oxo-D-homo-androsta-4, 17-diene-17a-carboxylate, chloromethyl 3-oxo-D-homo-androsta-4,17-diene-17a-carboxylate, methyl 3-oxo-D-homo-androsta-4,17-diene-17a-carboxylate, 3-oxo-D-homo-androsta-4,17-diene-17a-carboxaldehyde, 3-oxo-D-homo-androsta-4,17-diene-17a-carboxylic acid, 3-oxo-D-homo-androst-4-ene-17aβ-carboxaldehyde, 3-oxo-D-homo-androst-4-ene-17aβ-carboxamide, dimethyl 3-oxo-D-homo-androst-4-ene-17aβ-carboxamide, methyl 3-oxo-D-homo-androsta-4,17-diene-17a-carboxamide, (2′-diethylaminoethyl) 3-oxo-D-homo-androst-4-ene-17aβ-carboxamide hydrochloride, 3-oxo-D-homo-androst-4-ene-17aβ-carbonitrile.

8. Methyl 17a-methyl-3-oxo-D-homo-androsta-4,16-diene-17aβ-carboxylate, methyl 17a-methyl-3-oxo-D-homo-androst-4-ene-17aβ-carboxylate, methyl 17a-chloro-3-oxo-D-homo-androst-4-ene-17aβ-carboxylate, 17a-methyl-3-oxo-D-homo-androsta-4,16-diene-17aβ-carboxylic acid.

9. 4-Methyl-3-oxo-D-homo-androsta-4,17-diene-17a-carboxylic acid, 4-methyl-3-oxo-D-homo-androst-4-ene-17aβ-carboxylic acid, methyl 3-oxo-4-methyl-D-homo-androst-4-ene-17aβ-carboxylate, methyl 4-chloro-3-oxo-D-homo-androsta-4,17-diene-17a-carboxylate, methyl 4-chloro-3-oxo-D-homo-androst-4-ene-17aβ-carboxylate, methyl 4-methyl-3-oxo-D-homo-androsta-4,17-diene-17a-carboxylate, methyl 4-hydroxy-3-oxo-D-homo-androsta-4,17-diene-17a-carboxylate, methyl 4-hydroxy-3-oxo-D-homo-androst-4-ene-17aβ-carboxylate.

10. 4,5-Dihydrospiro[furan-2(3H),17′a(β1)-D-homoandrosta/4,16/dien]-3′-one and 4,5-dihydro-6′-methylene-spiro[furan-2(3H),17′a(β1)-D-homoandrosta(4,16)dien]-3′-one.

11. D-homosteroids of formula I as medicaments.

12. D-homosteroids of formula I as anti-androgens.

13. A process for the manufacture of D-homosteroids of formula I, characterized by

a) treating a D-homosteroid of the formula

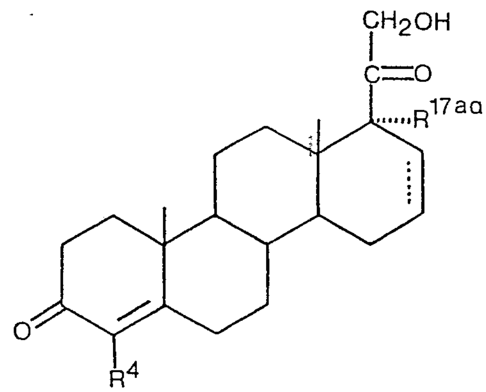

II

with an oxidation agent ; or

b) treating a D-homosteroid of the formula

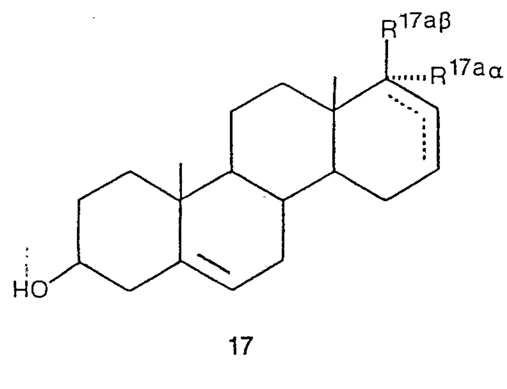

III

with an oxidation agent ; or

c) dehydrating a D-homosteroid of the formula

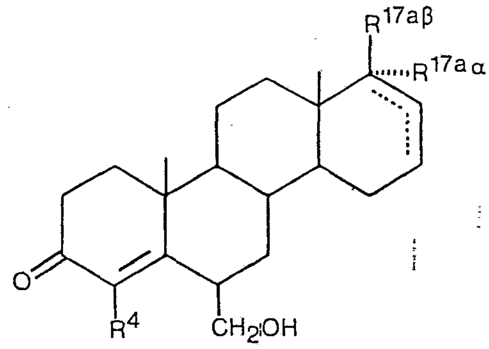

IV

or

d) cyclizing a D-homosteroid of the formula

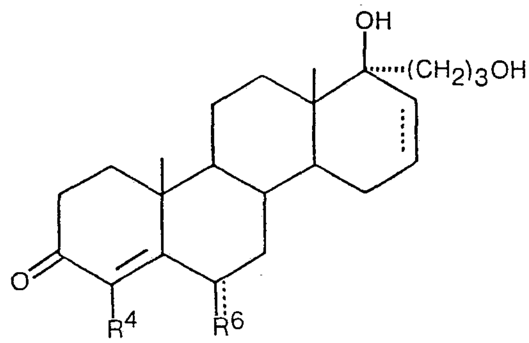

V

to the ether ; or

e) esterifying the carboxy group in a D-homosteroid of formula I in which $R^{17a\beta}$ is carboxy ; or

f) saponifying the esterified carboxy group in a D-homosteroid of formula I in which $R^{17a\beta}$ is esterified carboxy ; or

g) methylating, chlorinating or hydroxylating in the 4-position a D-homosteroid of formula I in which $R^4$ and $R^6$ are hydrogen ; or

h) amidating the carboxy group in a D-homosteroid of formula I in which $R^{17a\beta}$ is carboxy ; or

i) transforming the carbamoyl group in a D-homosteroid of formula I in which $R^{17a\beta}$ is carbamoyl into the cyano group.

14. Pharmaceutical preparations containing a D-homosteroid of formula I.

**Claims** (for the Contracting State AT)

1. A process for the manufacture of D-homosteroids of the formula

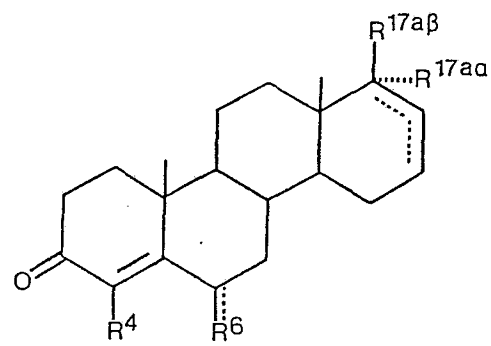

I

wherein $R^4$ signifies hydrogen, methyl, chlorine or hydroxy ; $R^6$ signifies hydrogen or methylene ; $R^{17a\alpha}$ signifies hydrogen, methyl or chlorine and $R^{17a\beta}$ signifies carboxy, esterified carboxy, cyano, formyl, carbamoyl, mono- or di-lower-alkyl-carbamoyl ; or $R^{17a\alpha}$ and $R^{17a\beta}$ together signify a spiroether ring of the formula

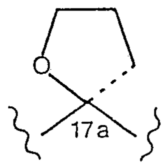

and wherein the dotted bonds in the D-ring represent an optional 16(17)- or 17(17a)-double bond. characterized by

a) treating a D-homosteroid of the formula

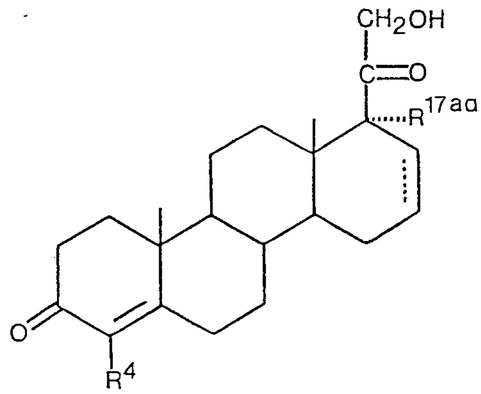

II

with an oxidation agent ; or

b) treating a D-homosteroid of the formula

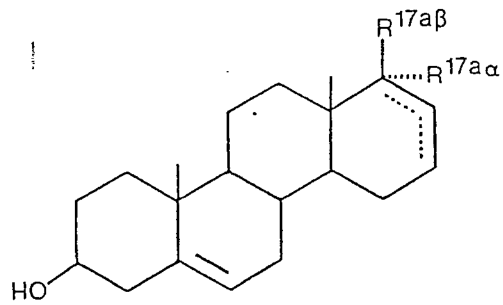

III

with an oxidation agent ; or

c) dehydrating a D-homosteroid of the formula

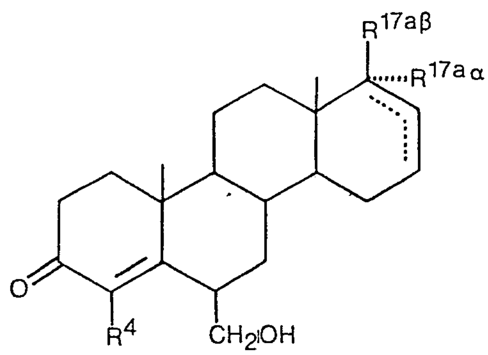

IV

or

d) cyclizing a D-homosteroid of the formula

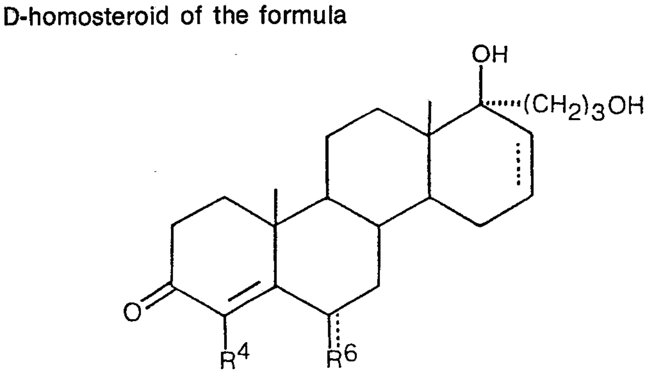

V

to the ether ; or

e) esterifying the carboxy group in a D-homosteroid of formula I in which $R^{17a\beta}$ is carboxy ; or

f) saponifying the esterified carboxy group in a D-homosteroid of formula I in which $R^{17a\beta}$ is esterified carboxy ; or

g) methylating, chlorinating or hydroxylating in the 4-position a D-homosteroid of formula I in which $R^4$ and $R^6$ are hydrogen ; or

h) amidating the carboxy group in a D-homosteroid of formula I in which $R^{17a\beta}$ is carboxy ; or

i) transforming the carbamoyl group in a D-homosteroid of formula I in which $R^{17a\beta}$ is carbamoyl into the cyano group.

2. A process according to claim 1, characterized in that D-homosteroids of formula I in which $R^4$, $R^6$ and $R^{17a\alpha}$ signify hydrogen are manufactured.

3. A process according to claim 1, characterized in that D-homosteroids of formula I in which $R^{17a\beta}$ signifies carboxy, lower-alkoxycarbonyl or cyano ; or $R^{17a\beta}$ together signify a spiroether ring of the formula

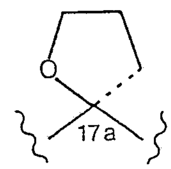

are manufactured.

4. A process according to claim 1, characterized in that D-homosteroids of formula I in which $R^4$, $R^6$ and $R^{17a\alpha}$ signify hydrogen and $R^{17a\beta}$ signifies carboxy or lower-alkoxycarbonyl and in which the D-ring is saturated are manufactured.

5. A process according to claim 1, characterized in that 3-oxo-D-homo-androst-4-ene-17aβ-carboxylic acid is manufactured.

6. A process according to claim 1, characterized in that methyl 3-oxo-D-homo-androst-4-ene-17aβ-carboxylate is manufactured.

7. A process according to claim 1, characterized in that isopropyl 3-oxo-D-homo-androsta-4,17-diene-17a-carboxylate, (2′-diethylamino)ethyl 3-oxo-D-homo-androsta-4, 17-diene-17a-carboxylate, chloromethyl 3-oxo-D-homo-androsta-4,17-diene-17a-carboxylate, methyl 3-oxo-D-homo-androsta-4,17-diene-17a-carboxylate, 3-oxo-D-homo-androsta-4,17-diene-17a-carboxaldehyde, 3-oxo-D-homo-androsta-4,17-diene-17a-carboxylic acid, 3-oxo-D-homo-androst-4-ene-17aβ-carboxaldehyde, 3-oxo-D-homo-androst-4-ene-17aβ-carboxamide, dimethyl 3-oxo-D-homo-androst-4-ene-17aβ-carboxamide, methyl 3-oxo-D-homo-androsta-4,17-diene-17a-carboxamide, (2′-diethylaminoethyl) 3-oxo-D-homo-androst-4-ene-17aβ-carboxamide hydrochloride or 3-oxo-D-homo-androst-4-ene-17aβ-carbonitrile is manufactured.

8. A process according to claim 1, characterized in that methyl 17a-methyl-3-oxo-D-homo-androsta-4,16-diene-17aβ-carboxylate, methyl 17a-methyl-3-oxo-D-homo-androst-4-ene-17aβ-carboxylate, methyl 17a-chloro-3-oxo-D-homo-androst-4-ene-17aβ-carboxylate or 17a-methyl-3-oxo-D-homoandrosta-4,16-diene-17aβ-carboxylic acid is manufactured.

9. A process according to claim 1, characterized in that 4-methyl-3-oxo-D-homo-androsta-4,17-diene-17a-carboxylic acid, 4-methyl-3-oxo-D-homo-androst-4-ene-17aβ-carboxylic acid, methyl 3-oxo-4-methyl-D-homo-androst-4-ene-17aβ-carboxylate, methyl 4-chloro-3-oxo-D-homo-androsta-4,17-diene-17a-carboxylate, methyl 4-chloro-3-oxo-D-homo-androst-4-ene-17aβ-carboxylate, methyl 4-methyl-3-oxo-D-homo-androsta-4,17-diene-17a-carboxylate, methyl 4-hydroxy-3-oxo-D-homo-androsta-4,17-diene-17a-carboxylate or methyl 4-hydroxy-3-oxo-D-homo-androst-4-ene-17aβ-carboxylate is manufactured.

10. A process according to claim 1, characterized in that 4,5-dihydrospiro[furan-2(3H),17′a(β1)-D-homoandrosta/4,16/dien]-3′-one or 4,5-dihydro-6′-methylenespiro [furan-2(3H),17′a(β1)-D-homoandrosta(4,16)dien]-3′-one is manufactured.

**Revendications** (pour les Etats contractants : BE, CH, DE, FR, GB, IT, LI, LU, NL, SE)

1. D-homostéroïdes de formule

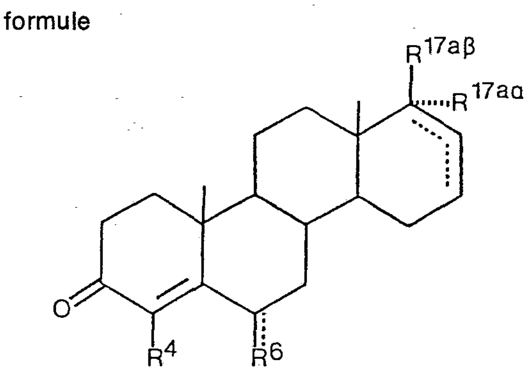

I

ou $R^4$ représente un hydrogène, un méthyle, un chlore ou un hydroxy ; $R^6$ représente un hydrogène ou un méthylène ; $R^{17a\alpha}$ représente un hydrogène, un méthyle ou un chlore et $R^{17a\beta}$ représente un carboxy, un carboxy estérifié, un cyano, un formyle, un carbamoyle, un mono- ou dialcoyle inférieur-carbamoyle ; ou bien où $R^{17a\alpha}$ et $R^{17a\beta}$ représentent ensemble un noyau spiroéther de formule

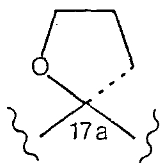

et où les liaisons pointillées dans le noyau D représentent une double liaison 16(17) ou 17(17a) facultative.

2. D-homostéroïdes selon la revendication 1, où $R^4$, $R^6$ et $R^{17a\alpha}$ représentent un hydrogène.

3. D-homostéroïdes selon la revendication 1, où $R^{17a\beta}$ représente un carboxy, un alcoxy inférieur-carbonyle ou un cyano ou $R^{17a\alpha}$ et $R^{17a\beta}$ représentent ensemble un noyau spiroéther de formule

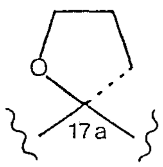

4. D-homostéroïdes selon la revendication 1, où $R^4$, $R^6$ et $R^{17a\alpha}$ représentent un hydrogène et $R^{17a\beta}$ représente un carboxy ou un alcoxy inférieur-carbonyle et où le noyau D est saturé.

5. Acide 3-oxo-D-homo-androst-4-ène-17aβ-carboxylique.

6. Ester méthylique de l'acide 3-oxo-D-homo-androst-4-ène-17aβ-carboxylique.

7. Isopropylester de l'acide 3-oxo-D-homo-androsta-4,17-diène-17a-carboxylique, (2'-diéthylamino)éthylester de l'acide 3-oxo-D-homo-androsta-4,17-diène-17a-carboxylique, chlorométhylester de l'acide 3-oxo-D-homo-androsta-4,17-diène-17a-carboxylique, ester méthylique de l'acide 3-oxo-D-homo-androsta-4,17-diène-17a-carboxylique, 3-oxo-D-homo-androsta-4,17-diène-17a-carboxaldéhyde, acide 3-oxo-D-homo-androsta-4,17-diène-17a-carboxylique, 3-oxo-D-homo-androst-4-ène-17aβ-carboxaldéhyde, amide de l'acide 3-oxo-D-homo-androst-4-ène-17aβ-carboxylique, diméthylamide de l'acide 3-oxo-D-homo-androst-4-ène-17a-carboxylique, méthylamide de l'acide 3-oxo-D-homo-androsta-4,17-diène-17a-carboxylique, chlorhydrate de (2'-diéthylaminoéthyl)amide de l'acide 3-oxo-D-homo-androst-4-ène-17aβ-carboxylique, nitrile de l'acide 3-oxo-D-homo-androst-4-ène-17aβ-carboxylique.

8. Ester méthylique de l'acide 17a-méthyl-3-oxo-D-homo-androsta-4,16-diène-17aβ-carboxylique, ester méthylique de l'acide 17a-méthyl-3-oxo-D-homo-androst-4-ène-17aβ-carboxylique, ester méthylique de l'acide 17a-chloro-3-oxo-D-homo-androst-4-ène-17aβ-carboxylique, acide 17a-méthyl-3-oxo-D-homo-androsta-4,16-diène-17aβ-carboxylique.

9. Acide 4-méthyl-3-oxo-D-homo-androsta-4,17-diène-17a-carboxylique, acide 4-méthyl-3-oxo-D-homo-androst-4-ène-17aβ-carboxylique, ester méthylique de l'acide 3-oxo-4-méthyl-D-homo-androst-4-ène-17aβ-carboxylique, ester méthylique de l'acide 4-chloro-3-oxo-D-homo-androsta-4,17-diène-17a-carboxylique, ester méthylique de l'acide 4-chloro-3-oxo-D-homo-androst-4-ène-16aβ-carboxylique, ester méthylique de l'acide 4-méthyl-3-oxo-D-homo-androsta-4,17-diène-17a-carboxylique, ester méthylique de l'acide 4-hydroxy-3-oxo-D-homo-androsta-4,17-diène-17a-carboxylique, ester méthylique de l'acide 4-hydroxy-3-oxo-D-homo-androst-4-ène-17aβ-carboxylique.

10. 4,5-dihydrospiro[furan-2(3H),17'a(β1)-D-homoandrosta/4,16/diène]-3'-one et 4,5-dihydro-6'-méthylènespiro-[furan-2(3H),17'a(β1))-D-homoandrosta(4,16)diène]-3'-one.

11. D-homostéroïdes de formule I comme médicaments.

12. D-homostéroïdes de formule I comme anti-androgènes.

13. Procédé de préparation de D-homostéroïdes de formule I, caractérisé en ce que

a) on traite un D-homostéroïde de formule

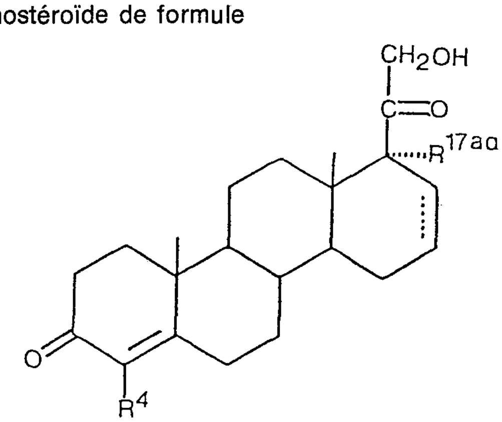

II

avec un oxydant ; ou

b) on traite un D-homostéroïde de formule

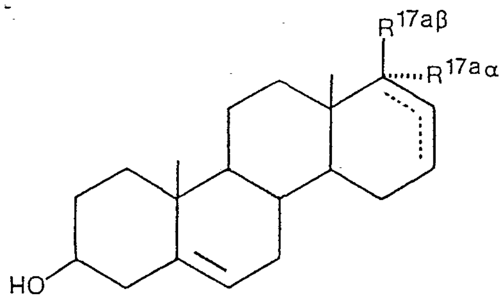

III

avec un oxydant ; ou

c) on déshydrate un D-homostéroïde de formule

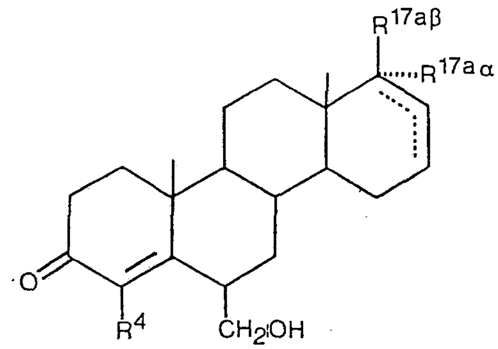

IV

ou

d) on cyclise un D-homostéroïde de formule

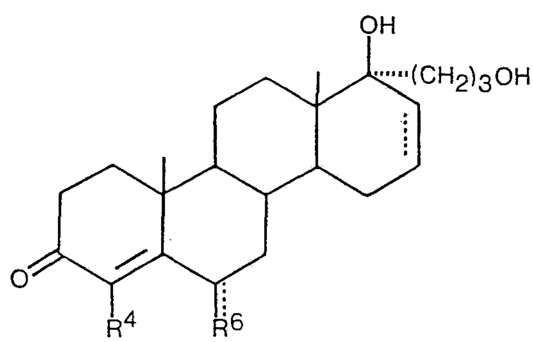

V

pour donner un éther ; ou

e) dans un D-homostéroïde de formule I, où $R^{17a\beta}$ est un carboxy, on estérifie le groupe carboxy ; ou

f) dans un D-homostéroïde de formule I, où $R^{17a\beta}$ est un carboxy estérifié, on saponifie le groupe carboxy estérifié ; ou

g) on méthyle, on chlore ou on hydroxyle un D-homostéroïde de formule I, où $R^4$ et $R^6$ sont des hydrogènes, en position 4 ; ou

h) dans un D-homostéroïde de formule I ou $R^{17a\beta}$ est un carboxy, on amide le groupe carboxy ; ou

i) dans un D-homostéroïde de formule I où $R^{17a\beta}$ est un carbamoyle, on transforme le groupe carbamoyle en le groupe cyano.

14. Préparations pharmaceutiques contenant un D-homostéroïde de formule I.

**Revendications** (pour l'Etat contractant AT)

1. Procédé de préparation de D-homostéroïdes de formule

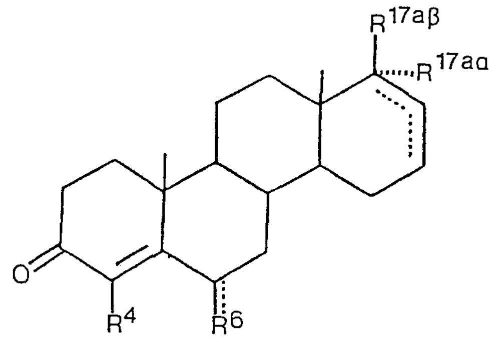

I

où $R^4$ représente un hydrogène, un méthyle, un chlore ou un hydroxy ; $R^6$ représente un hydrogène ou un méthylène ; $R^{17a\alpha}$ représente un hydrogène, un méthyle ou un chlore et $R^{17a\beta}$ représente un carboxy, un carboxy estérifié, un cyano, un formyle, un carbamoyle, un mono- ou di-alcoyle inférieur-carbamoyle ; ou $R^{17a\alpha}$ et $R^{17a\beta}$ représentent ensemble un noyau spiroéther de formule

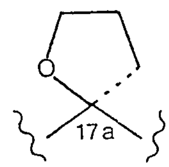

et où les liaisons pointillées dans le noyau D représentent une double liaison 16(17) ou 17(17a) facultative, caractérisé en ce que

a) on traite un D-homostéroïde de formule

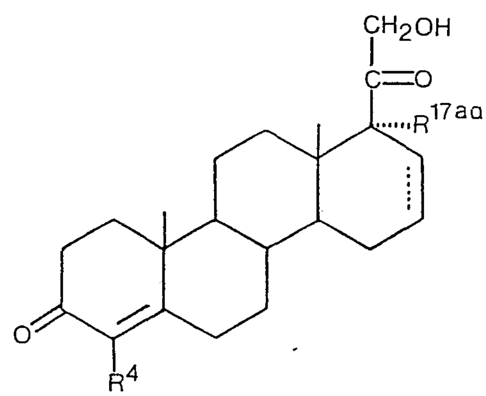

II

avec un oxydant ; ou

b) on traite un D-homostéroïde de formule

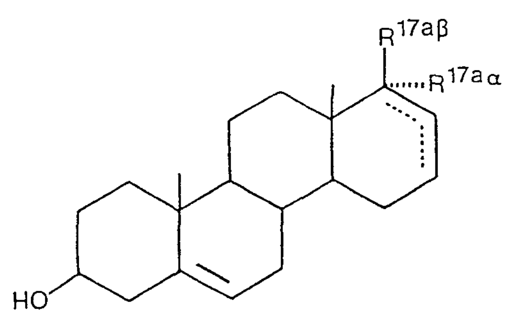

III

avec un oxydant ; ou

c) on déshydrate un D-homostéroïde de formule

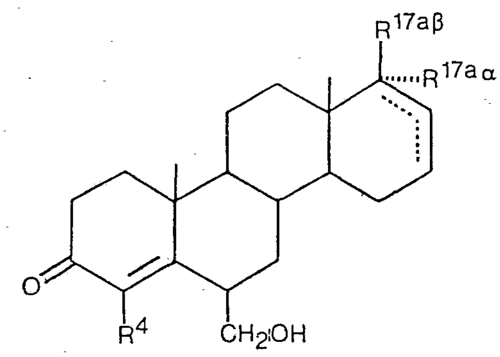

IV

ou

d) on cyclise un D-homostéroïde de formule

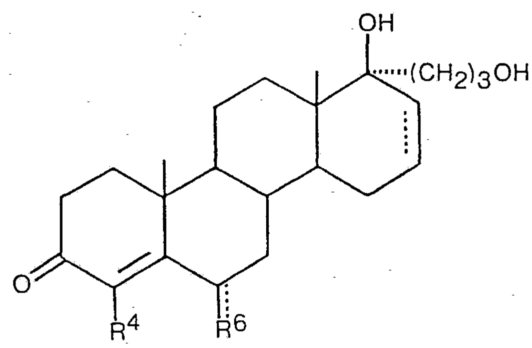

V

pour donner un éther ; ou

e) dans un D-homostéroïde de formule I où $R^{17a\beta}$ est un carboxy on estérifie le groupe carboxy ; ou

f) dans un D-homostéroïde de formule I où $R^{17a\beta}$ est un carboxy estérifié, on saponifie le groupe carboxy estérifié ; ou

g) on méthyle, on chlore ou on hydroxyle en position 4 un D-homostéroïde de formule I, où $R^4$ et $R^6$ sont des hydrogènes ; ou

h) dans un D-homostéroïde de formule I où $R^{17a\beta}$ est un carboxy, on amide le groupe carboxy ; ou

i) dans un D-homostéroïde de formule I où $R^{17a\beta}$ est un carbamoyle, on transforme le groupe carbamoyle en le groupe cyano.

2. Procédé selon la revendication 1, caractérisé en ce qu'on prépare des D-homostéroïdes de formule I où $R^4$, $R^6$ et $R^{17a\alpha}$ représentent un hydrogène.

3. Procédé selon la revendication 1, caractérisé en ce qu'on prépare des D-homostéroïdes de formule I où $R^{17a\beta}$ représente un carboxy, un alcoxy inférieur-carbonyle ou un cyano ; ou bien où $R^{17a\alpha}$ et $R^{17a\beta}$ représentent ensemble un noyau spiroéther de formule

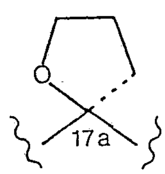

4. Procédé selon la revendication 1, caractérisé en ce qu'on prépare des D-homostéroïdes de formule I où $R^4$, $R^6$ et $R^{17a\alpha}$ représentent un hydrogène et $R^{17a\beta}$ représentent un carboxy ou un alcoxy inférieur-carbonyle et où le noyau D est saturé.

5. Procédé selon la revendication 1, caractérisé en ce qu'on prépare l'acide 3-oxo-D-homo-androst-4-ène-17aβ-carboxylique.

6. Procédé selon la revendication 1, caractérisé en ce qu'on prépare l'ester méthylique de l'acide 3-oxo-D-homo-androst-4-ène-17aβ-carboxylique.

7. Procédé selon la revendication 1, caractérisé en ce qu'on prépare l'isopropylester de l'acide 3-oxo-D-homo-androsta-4,17-diène-17a-carboxylique, le (2'-diéthyl-amino)éthylester de l'acide 3-oxo-D-homo-androsta-4,17-diène-17a-carboxylique, le chlorométhylester de l'acide 3-oxo-D-homo-androsta-4,17-diène-17a-carboxylique, le méthylester de l'acide 3-oxo-D-homo-androsta-4,17-diène-17a-carboxylique, le 3-oxo-D-homo-androsta-4,17-diène-17a-carboxaldéhyde, l'acide 3-oxo-D-homo-androsta-4,17-

diène-17a-carboxylique, le 3-oxo-D-homo-androst-4-ène-17aβ-carboxaldéhyde, l'amide de l'acide 3-oxo-D-homo-androst-4-ène-17aβ-carboxylique, le diméthylamide de l'acide 3-oxo-D-homo-androst-4-ène-16aβ-carboxylique, le méthylamide de l'acide 3-oxo-D-homo-androsta-4,17-diène-17a-carboxylique, le chlorhydrate de (2'-diéthylaminoéthyl) amide de l'acide 3-oxo-D-homo-androst-4-ène-17aβ-carboxylique ou le nitrile de l'acide 3-oxo-D-homo-androst-4-ène-17aβ-carboxylique.

8. Procédé selon la revendication 1, caractérisé en ce qu'on prépare l'ester méthylique de l'acide 17a-méthyl-3-oxo-D-homo-androsta-4,16-diène-17aβ-carboxylique, l'ester méthylique de l'acide 17a-méthyl-3-oxo-D-homo-androst-4-ène-17aβ-carboxylique, l'ester méthylique de l'acide 17a-chloro-3-oxo-D-homo-androst-4-ène-17aβ-carboxylique ou l'acide 17a-méthyl-3-oxo-D-homo-androsta-4,16-diène-17aβ-carboxylique.

9. Procédé selon la revendication 1, caractérisé en ce qu'on prépare l'acide 4-méthyl-3-oxo-D-homo-androsta-4,17-diène-17a-carboxylique, l'acide 4-méthyl-3-oxo-D-homo-androst-4-ène-17aβ-carboxylique, l'ester méthylique de l'acide 3-oxo-4-méthyl-D-homo-androst-4-ène-17aβ-carboxylique, l'ester méthylique de l'acide 4-chloro-3-oxo-D-homo-androsta-4,17-diène-17a-carboxylique, l'ester méthylique de l'acide 4-chloro-3-oxo-D-homo-androst-4-ène-17aβ-carboxylique, l'ester méthylique de l'acide 4-méthyl-3-oxo-D-homo-androsta-4,17-diène-17a-carboxylique, l'ester méthylique de l'acide 4-hydroxy-3-oxo-D-homo-androsta-4,17-diène-17a-carboxylique, ou l'ester méthylique de l'acide 4-hydroxy-3-oxo-D-homo-androst-4-ène-17aβ-carboxylique.

10. Procédé selon la revendication 1, caractérisé en ce qu'on prépare la 4,5-dihydrospiro[furan-2(3H),17'a(β1)-D-homoandrosta/4,16/diène]-3'-one ou la 4,5-dihydro-6'-méthylènespiro[furan-2(3H),17'a(β1)-D-homoandrosta-(4,16)diène]-3'-one.